Europäisches Patentamt

European Patent Office

Office européen des brevets

(11) Publication number: **0 326 907**
**A1**

# EUROPEAN PATENT APPLICATION

(21) Application number: 89101162.9

(51) Int. Cl.⁴: **C12N 15/00**

(22) Date of filing: 24.01.89

The applicant has filed a statement in accordance with Rule 28 (4) EPC (issue of a sample only to an expert). Accession number(s) of the deposit(s): FERM BP-1281, BP-1593, BP-1645, BP-2009, BP-2010, BP-2011, BP-2012, BP-2013, BP-2203 & IFO-50142, 50143, 50144, 50145.

(30) Priority: 26.01.88 JP 16260/88
19.08.88 JP 206968/88
20.09.88 JP 235842/88

(43) Date of publication of application:
09.08.89 Bulletin 89/32

(84) Designated Contracting States:
AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

(72) Inventor: Senoo, Masaharu
B-212, 744-1 Nishi-izumigaoka 2-chome
Toyonaka Osaka 560(JP)
Inventor: Sasada, Reiko
C1-405, 2 Takenodai
Nagaokakyo Kyoto 617(JP)
Inventor: Kurokawa, Tsutomu
1-50, Suimeidai 1-chome
Kawanishi Hyogo 666-01(JP)
Inventor: Igarashi, Koichi
66-3, Shimogamo-miyazakicho
Sakyo-ku Kyoto 606(JP)

(74) Representative: von Kreisler, Alek,
Dipl.-Chem. et al
Patentanwälte Von Kreisler-Selting-Werner
Deichmannhaus am Hauptbahnhof
D-5000 Köln 1(DE)

(54) Polypeptide, DNA and its use.

(57) A mutein of basic fibroblast growth factor (bFGF) which lacks 7 to 46 amino acids of the constituent amino acids of bFGF from carboxyl terminal possesses excellent fibroblast growth promoting activities, vasoendothelial cell growth promoting activities and angiogenic activities. It is very stable in living cells. The mutein of the present invention therefore function well as a healing promoter for burns etc., therapeutic drug for thrombosis, arteriosclerosis etc., and cell cultivation promoter.

EP 0 326 907 A1

## POLYPEPTIDE, DNA AND ITS USE

The present invention relates to muteins of basic fibroblast growth factors (hereinafter also briefly referred to as bFGF), a recombinant DNA segment having a base sequence which codes for a mutein of bFGF and its use.

bFGF is a basic polypeptide hormone having a molecular weight of about 17,000 secreted mainly by the pituitary gland, and was first separated as a factor exhibiting strong growth promoting action on fibroblasts such as BALB/c3T3 [D. Gospodarowicz; Nature, *249*, 123 (1974)]. Thereafter, however, it was proven that it exhibits growth promoting action on almost all cells deriving from mesoblast [D. Gospodarowicz et al.; National Cancer Institute Monograph, *48*, 109 (1978)]. Specifically, the angiogenic action of bFGF, together with its cell growth promoting action, suggests of a potential for the application thereof as a therapeutic drug for traumas and as a preventive and therapeutic drug for thrombosis, arteriosclerosis, etc.

Bovine bFGF was first described in the Proceedings of the National Academy of Sciences of the United States of America, Vol. 82, pp. 6507 (1985). In Science, *233*, 545 (1986), there were disclosed the bovine bFGF-constituent amino acids deduced from a clone which was produced by cloning cDNA of human bFGF.

As for human bFGF, Biochemical and Biophysical Research Communications, Vol. 135, p. 541 (1986) stated that human bFGF was extracted from human brain.

In European Molecular Biology Organization (EMBO) Journal, Vol 5, p. 2523 (1986) and PCT International Publication No. WO/87/01728, state that human bFGF-constituent amino acids were deductively specified on the basis of a clone which was produced by cloning cDNA of human bFGF using bovine bFGF as a probe.

In addition, FEBS Letters, *213*, 189 (1987), describes the production of human bFGF by cultivation of a transformant obtained by cloning cDNA of human bFGF.

Comparing human bFGF and bovine bFGF, the human one has *Thr* for the 112-position, while the bovine one, *Ser*; the human one *Ser* for the 128-position, while the bovine one has *Pro*. (In this case, the N-terminal Pro is determined as the lst amino acid.).

The present inventors postulated that modifications in the amino acid sequence improved stability, productivity in a cell and cell proliferating activity per molecule of bFGF, and, in addition, its unidentified bioactivities were potentiated.

Particularly, bFGF is known to induce cellular morphological changes like those found in malignant cells when expressed in excess in animal cells or expressed in the presence of the signal peptide of a foreign protein as an adduct thereto [R. Sasada, Molecular and Cellular Biology, *8*, 588 (1988); S. Rogelj, Nature, *331*, 173 (1988)].

From the viewpoint of application of bFGF to pharmaceuticals etc., it is desired that a bFGF mutein having none of the above-mentioned undesirable properties will be produced.

In light of these circumstances, the present inventors investigated various bFGF muteins, and conjectured that amino acid-lacking muteins may be efficient.

In addition, biologically active proteins microbiologically produced by DNA recombination technique contain cysteine residues, which are not essential to their activities but which may form undesirable intermolecular or intramolecular linkages.

In the course of a production of bFGF by recombinant DNA technique, heterogeneous conformations were found in *Escherichia coli* extract containing a high concentration of bFGF. These conformations are thought to have been produced due to random intra-or inter-molecular disulfide bridging, and cause difficulties in bFGF purification and reduce its recovery.

The present inventors decided it would be useful if one could modify microbiologically produced bioactive proteins such as recombinant bFGF so that, while their activities are not affected adversely, their capabilities of forming such intermolecular bridges and intramolecular linkages as will result in the formation of undesirable tertiary structures (e.g., conformations which lower the activity of the protein) are reduced or eliminated.

The present inventors constructed muteins of bFGF lacking constituent amino acids by DNA recombination and site-directed mutagenesis, and made investigations to improve their stability, upgrade their intracellular productivities and activities, and describe changes in their bioactivities; the present inventors thus found a mutein which serves these purposes, particularly exhibiting excellent capability of inducing changes like those found in malignant cells and stability to acidic conditions, and made further investigations based on this finding to develop the present invention.

The present invention involves:

(1) a mutein of bFGF which lacks 7 to 46 amino acid residues from the carboxyl terminal;

(2) a mutein as the above (1), which further at least one constituent amino acid may be replaced by another amino acid and/or at least one amino acid may be added to the amino terminal,

(3) a DNA having a base sequence which codes for the above-mentioned mutein (1) or (2),

(4) a transformant harboring a vector containing a recombinant DNA having a base sequence which codes for the above-mentioned mutein (1) or (2), and

(5) a method for producing the above-mentioned mutein (1) or (2), which comprises subjecting a transformant harboring a vector containing a recombinant DNA having a base sequence which codes for said mutein to cultivation in a culture medium to produce and accumulate said mutein in the culture broth

In said mutein (2), both the above-mentioned replacement and addition may be underwent.

For bFGF, any bFGF can be included, as long as it derives from a warm-blooded mammal.

Typical examples thereof include human, bovine and murine bFGFs.

Polypeptides which include the amino acid sequence represented as follows:

Phe-Phe-Leu-Arg-Ile-His-Pro-Asp-Gly-Arg-Val-Asp-Gly-Val-Arg-Glu-Lys-Ser-Asp-Pro     [I]

are preferable.

More preferably, the polypeptides are represented by the formula:

Pro-Ala-Leu-Pro-Glu-Asp-Gly-Gly-Ser-Gly-Ala-Phe-Pro-Pro-Gly-His-Phe-Lys-Asp-Pro-Lys-Arg-Leu-Tyr-Cys-
Lys-Asn-Gly-Gly-Phe-Phe-Leu-Arg-Ile-His-Pro-Asp-Gly-Arg-Val-Asp-Gly-Val-Arg-Glu-Lys-Ser-Asp-Pro-His-
Ile-Lys-Leu-Gln-Leu-Gln-Ala-Glu-Glu-Arg-Gly-Val-Val-Ser-Ile-Lys-Gly-Val-Cys-Ala-Asn-Arg-Tyr-Leu-Ala-Met-
Lys-Glu-Asp-Gly-Arg-Leu-Leu-Ala-Ser-Lys-Cys-Val-Thr-Asp-Glu-Cys-Phe-Phe-Phe-Glu-Arg-Leu-Glu-Ser-Asn-
Asn-Tyr-Asn-Thr-Tyr-Arg-Ser-Arg-Lys-Tyr-X- Ser-Trp-Tyr-Val-Ala-Leu-Lys-Arg-Thr-Gly-Gln-Tyr-Lys-Leu-Gly-
Y-Lys-Thr-Gly-Pro-Gly-Gln-Lys-Ala-Ile-Leu-Phe-Leu-Pro-Met-Ser-Ala-Lys-Ser,     [II]

in which X represents *Thr* or *Ser*; when X is *Thr*, Y represents *Ser*, and when X is *Ser*, Y represents *Pro*

Polypeptides containing the amino acid sequence:

Pro-Ala-Leu-Pro-Glu-Asp-Gly-Gly-Gly-Ala-Phe-Pro-Pro-Gly-His-Phe-Lys-Asp-Pro-Lys-Arg-Leu-Tyr-Cys-Lys-
Asn-Gly-Gly-Phe-Phe-Leu-Arg-Ile-His-Pro-Asp-Gly-Arg-Val-Asp-Gly-Val-Arg-Glu-Lys-Ser-Asp-Pro-His-Val-
Lys-Leu-Gln-Leu-Gln-Ala-Glu-Glu-Arg-Gly-Val-Val-Ser-Ile-Lys-Gly-Val-Cys-Ala-Asn-Arg-Tyr-Leu-Ala-Met-Lys- .
Glu-Asp-Gly-Arg-Leu-Leu-Ala-Ser-Lys-Cys-Val-Thr-Glu-Glu-Cys-Phe-Phe-Phe-Glu-Arg-Leu-Glu-Ser-Asn-Asn-
Tyr-Asn-Thr-Tyr-Arg-Ser-Arg-Lys-Tyr-Ser-Ser-Trp-Tyr-Val-Ala-Leu-Lys-Arg-Thr-Gly-Gln-Tyr-Lys-Leu-Gly-
Ser-Lys-Thr-Gly-Pro-Gly-Gln-Lys-Ala-Ile-Leu-Phe-Leu-Pro-Met-Ser-Ala-Lys-Ser     [III]

are even more preferable.

The mutein of the present invention is one which lacks 7 to 46 constituent amino acids of the original bFGF peptide or protein from the carboxyl terminal.

As examples of the preferred amino acid sequences to be lacked, mention may be made of the amino acid sequences respectively starting at rhbFGF amino acid Nos. 102, 106, 115, 119, 124, 130, and 138.

These amino acid sequences may be replaced by another amino acid.

Examples of the constituent amino acid to be replaced in the mutein in which at least one constituent amino acid is replaced by other amino acid include cysteine and others; cysteine is preferred. As examples of the constituent amino acid to be replaced, other than cysteine, mention may be made of aspartic acid, arginine, glycine, valine, and isoleucine.

When the constituent amino acid before substitution is cysteine, the substituted amino acids are preferably, for example, neutral amino acids. As specific examples of such neutral amino acids, mention may be made of glycine, valine, alanine, leucine, isoleucine, tyrosine, phenylalanine, histidine, tryptophan, serine, threonine and methionine. Serine and threonine are preferable.

When the constituent amino acid before substitution is other than cysteine, the substituted amino acids are selected from amino acids which are different from the amino acid before substitution in a property such as hydrophilicity, hydrophobicity or electric charge.

When the constituent amino acid before substitution is aspartic acid, examples of the substituted amino acids include asparagine, threonine, valine, phenylalanine and arginine; asparagine and arginine are preferable.

When the constituent amino acid before substitution is arginine, examples of the substituted amino acids include glutamine, threonine, leucine, phenylalanine and aspartic acid; glutamine is preferable.

When the constituent amino acid before substitution is glycine, examples of the substituted amino acids include threonine, leucine, phenilalanine, serine, glutamic acid, and arginine; threonine is preferable.

When the constituent amino acid before substitution is serine, examples of the substituted amino acids include methionine, alanine, leucine, cysteine, glutamine, arginine and aspartic acid; methionine is preferable.

When the constituent amino acid before substitution is valine, examples of the substituted amino acids include serine, leucine, proline, glycine, lysine, and aspartic acid; serine is preferable.

When the constituent amino acid to be replaced is isoleucine, examples of the amino acid as the replacement include serine, glycine, valine, alanine, leucine, tyrosine, phenylalanine, histidine, tryptophan, and methionine; serine is preferred

The constituent amino acid before substitution are preferably aspartic acid, arginine, glycine, serine and valine.

The substituted amino acid is preferably asparagine, glutamine, arginine, threonine, methionine, serine, and leucine.

The embodiment on the substitution in the mutein wherein there is a substitution of serine for cysteine (i.e. cysteine is replaced by serine) is most preferred.

The at least one amino acid in the mutein which has at least one amino acid added to bFGF excludes methionine deriving from initiation codon used for peptide expression and signal peptide.

The number of added amino acids is at least 1, but it may be any one, as long as bFGF characteristics are not lost. Preferably the amino acids added should include some or all of the amino acid sequences of proteins which have homology with bFGF and which exhibit activities similar to those of bFGF.

As examples of such added amino acids, mention may be made of a part or all of the amino acid sequences of proteins such as acidic fibroblast growth factors (aFGF), interleukin 1-α (IL1-α), interleukin 1-β(IL1-β), and a protein which is coded by int-2 in oncogenes.

Such aFGFs include those deriving from humans and those deriving from bovines. Such IL1-αs and IL1-βs include those deriving from humans.

Amino acid sequences of such bovine aFGFs include:

NH₂-Phe-Asn-Leu-Pro-Leu-Gly-Asn-Tyr-Lys-Lys-Pro-Lys-Leu-Leu-Tyr-Cys-Ser-Asn-Gly-Gly-Tyr-Phe-Leu-Arg-Ile-Leu-Pro-Asp-Gly-Thr-Val-Asp-Gly-Thr-Lys-Asp-Arg-Ser-Asp-Gln-His-Ile-Gln-Leu-Gln-Leu-Cys-Ala-Glu-Ser-Ile-Gly-Glu-Val-Tyr-Ile-Lys-Ser-Thr-Glu-Thr-Gly-Gln-Phe-Leu-Ala-Met-Asp-Thr-Asp-Gly-Leu-Leu-Tyr-Gly-Ser-Gln-Thr-Pro-Asn-Glu-Glu-Cys-Leu-Phe-Leu-Glu-Arg-Leu-Glu-Glu-Asn-His-Tyr-Asn-Thr-Tyr-Ile-Ser-Lys-Lys-His-Ala-Glu-Lys-His-Trp-Phe-Val-Gly-Leu-Lys-Lys-Asn-Gly-Arg-Ser-Lys-Leu-Gly-Pro-Arg-Thr-His-Phe-Gly-Gln-Lys-Ala-Ile-Leu-Phe-Leu-Pro-Leu-Pro-Val-Ser-Ser-Asp-COOH

Amino acid sequences of human IL1-α include:

NH₂-Met-Ala-Lys-Val-Pro-Asp-Met-Phe-Glu-Asp-Leu-Lys-Asn-Cys-Tyr-Ser-Glu-Asn-Glu-Glu-Asp-Ser-Ser-Ser-Ile-Asp-His-Leu-Ser-Leu-Asn-Gln-Lys-Ser-Phe-Tyr-His-Val-Ser-Tyr-Gly-Pro-Leu-His-Glu-Gly-Cys-Met-Asp-Gln-Ser-Val-Ser-Leu-Ser-Ile-Ser-Glu-Thr-Ser-Lys-Thr-Ser-Lys-Leu-Thr-Phe-Lys-Glu-Ser-Met-Val-Val-Val-Ala-Thr-Asn-Gly-Lys-Val-Leu-Lys-Lys-Arg-Arg-Leu-Ser-Leu-Ser-Gln-Ser-Ile-Thr-Asp-Asp-Asp-Leu-Glu-Ala-Ile-Ala-Asn-Asp-Ser-Glu-Glu-Glu-Ile-Ile

$$-\boxed{\begin{matrix} \text{Lys} \\ \text{Asn} \end{matrix}}-$$

Pro-Arg-Ser-Ala-Pro-Phe-Ser-Phe-Leu-Ser-Asn-Val-Lys-Tyr-Asn-Phe-Met-Arg-Ile-Ile-Lys-Tyr-Glu-Phe-Ile-Leu-Asn-Asp-Ala-Leu-Asn-Gln-Ser-Ile-Ile-Arg-Ala-Asn-Asp-Gln-Tyr-Leu-Thr-Ala-Ala-Ala-Leu-His-Asn-Leu-Asp-Glu-Ala-Val-Lys-Phe-Asp-Met-Gly-Ala-Tyr-Lys-Ser-Ser-Lys-Asp-Asp-Ala-Lys-Ile-Thr-Val-Ile-Leu-Arg-Ile-Ser-Lys-Thr-Gln-Leu-Tyr-Val-Thr-Ala-Gln-Asp-Glu-Asp-Gln-Pro-Val-Leu-Leu-Lys-Glu-Met-Pro-Glu-Ile-Pro-Lys-Thr-Ile-Thr-Gly-Ser-Glu-Thr-Asn-Leu-Leu-Phe-Phe-Trp-Glu-Thr-His-Gly-Thr-Lys-Asn-Tyr-Phe-Thr-Ser-Val-Ala-His-Pro-Asn-Leu-Phe-Ile-Ala-Thr-Lys-Gln-Asp-Tyr-Trp-Val-Cys-Leu-     Ala-Gly-Gly-Pro-Pro-Ser-Ile-Thr-Asp-Phe-Gln-Ile-Leu-Glu-Asn-Gln-Ala-COOH

Amino acid sequences of human IL1-β include:

NH₂-Met-Ala-Glu-Val-Pro-Glu-Leu-Ala-Ser-Glu-Met-Met-Ala-Tyr-Tyr-Ser-Gly-Asn-Glu-Asp-Asp-Leu-Phe-Phe-Glu-Ala-Asp-Gly-Pro-Lys-Gln-Met-Lys-Cys-Ser-Phe-Gln-Asp-Leu-Asp-Leu-Cys-Pro-Leu-Asp-Gly-Gly-Ile-Gln-Leu-Arg-Ile-Ser-Asp-His-His-Tyr-Ser-Lys-Gly-Phe-Arg-Gln-Ala-Ala-Ser-Val-Val-Val-Ala-Met-Asp-Lys-Leu-Arg-Lys-Met-Leu-Val-Pro-Cys-Pro-Gln-Thr-Phe-Gln-Glu-Asn-Asp-Leu-Ser-Thr-Phe-Phe-Pro-Phe-Ile-Phe-Glu-Glu-Glu-Pro-Ile-Phe-Phe-Asp-Thr-Trp-Asp-Asn-Glu-Ala-Tyr-Val-His-Asp-Ala-Pro-Val-Arg-Ser-Leu-Asn-Cys-Thr-Leu-Arg-Asp-Ser-Gln-Gln-Lys-Ser-Leu-Val-Met-Ser-Gly-Pro-Tyr-Glu-Leu-Lys-Ala-Leu-His-Leu-Gln-Gly-Gln-Asp-Met-Glu-Gln-Gln-Val-Val-Phe-Ser-Met-Ser-Phe-Val-Gln-Gly-Glu-Glu-Ser-Asn-Asp-Lys-Ile-Pro-Val-Ala-Leu-Gly-Leu-Lys-Glu-Lys-Asn-Leu-Tyr-Leu-Ser-Cys-Val-Leu-Lys-Asp-Asp-Lys-Pro-Thr-Leu-Gln-Leu-Glu-Ser-Val-Asp-Pro-Lys-Asn-Tyr-Pro-Lys-Lys-Lys-Met-Glu-Lys-Arg-Phe-Val-Phe-Asn-Lys-Ile-Glu-Ile-Asn-Asn-Lys-Leu-Glu-Phe-Glu-Ser-Ala-Gln-Phe-Pro-Asn-Trp-Tyr-Ile-Ser-Thr-Ser-Gln-Ala-Glu-Asn-Met-Pro-Val-Phe-Leu-Gly-Gly-Thr-Lys-Gly-Gly-Gln-Asp-Ile-Thr-Asp-Phe-Thr-Met-Gln-Phe-Val-Ser-Ser-COOH

Amino acid sequences encoded to int-2 include: NH$_2$-Met-Gly-Leu-Ile-Trp-Leu-Leu-Leu-Leu-Ser-Leu-Leu-Glu-Pro-Ser-Trp-Pro-Thr-Thr-Gly-Pro-Gly-Thr-Arg-Leu-Arg-Arg-Asp-Ala-Gly-Gly-Arg-Gly-Gly-Val-Tyr-Glu-His-Leu-Gly-Gly-Ala-Pro-Arg-Arg-Arg-Lys-Leu-Tyr-Cys-Ala-Thr-Lys-Tyr-His-Leu-Gln-Leu-His-Pro-Ser-Gly-Arg-Val-Asn-Gly-Ser-Leu-Glu-Asn-Ser-Ala-Tyr-Ser-Ile-Leu-Glu-Ile-Thr-Ala-Val-Glu-Val-Gly-Val-Val-Ala-Ile-Lys-Gly-Leu-Phe-Ser-Gly-Arg-Tyr-Leu-Ala-Met-Asn-Lys-Arg-Gly-Arg-Leu-Tyr-Ala-Ser-Asp-His-Tyr-Asn-Ala-Glu-Cys-Glu-Phe-Val-Glu-Arg-Ile-His-Glu-Leu-Gly-Tyr-Asn-Thr-Tyr-Ala-Ser-Arg-Leu-Tyr-Arg-Thr-Gly-Ser-Ser-Gly-Pro-Gly-Ala-Gln-Arg-Gln-Pro-Gly-Ala-Gln-Arg-Pro-Trp-Tyr-Val-Ser-Val-Asn-Gly-Lys-Gly-Arg-Pro-Arg-Arg-Gly-Phe-Lys-Thr-Arg-Arg-Thr-Gln-Lys-Ser-Ser-Leu-Phe-Leu-Pro-Arg-Val-Leu-Gly-His-Lys-Asp-His-Glu-Met-Val-Arg-Leu-Leu-Gln-Ser-Ser-Gln-Pro-Arg-Ala-Pro-Gly-Glu-Gly-Ser-Gln-Pro-Arg-Gln-Arg-Arg-Gln-Lys-Lys-Gln-Ser-Pro-Gly-Asp-His-Gly-Lys-    Met-Glu-Thr-Leu-Ser-Thr-Arg-Ala-Thr-Pro-Ser-Thr-Gln-Leu-His-Thr-Gly-Gly-Leu-Ala-Val-Ala-COOH

In addition to conventional DNA recombination technique site-directed mutagenesis is employed to produce the mutein of the present invention. The said technique is well-known, and it is described in Genetic Engineering, Lather, R. F. and Lecoq, J. P., Academic Press, pp. 31 to 50 (1983). Mutagenesis directed to oligonucleotide is described in Genetic Engineering: Principles and Methods, Smith, M. and Gillam, S., Plenum Press, Vol. 3, pp. 1 to 32 (1981).

The production of the structural gene which encodes the mutein of the present invention is, for example, carried out by:

(a) hybridizing with a mutagenic oligonucleotide primer a single-stranded DNA comprising 1 strand of the structural gene of bFGF,

(b) elongating the primer using DNA polymerase to form a mutational heteroduplex, and

(c) replicating this mutational heteroduplex.

The size of oligonucleotide primer used depends upon conditions necessary for stable hybridization of the primer to the gene region to which mutation is to be introduced Limitations in currently available methods of oligonucleotide synthesis also affect which size is preferable. The factors to be considered in designing the oligonucleotide intended for the use for mutagenesis directed by the oligonucleotide (e.g., the overall size of the nucleotide and the size of the mismatching portion at the mutation site) are well known and, for example, are described by Smith, M. and Gillam, S. in the above-mentioned literature. In general, the overall length of the oligonucleotide is adjusted to such length that stable and unique hybridization at the mutation site is optimized, and the extensions between the mutation site and the 5′- and 3′-terminals are provided with sufficient sizes to prevent mutation induced by the exonuclease activity of DNA polymerase.

The oligonucleotides used for mutagenesis in accordance with the present invention normally contain some 12 to 24 bases, preferably 14 to 20 bases, and more preferably 14 to 18 bases. These normally contain at least about 3 base 3′-terminal of the codons to be changed.

For the purpose of obtaining, for example, a mutein having an added amino acid, a mutagenic bFGF gene is produced by synthesizing the gene which encodes the amino acid sequence to be added, and, directly or after fragmentation by digestion with an appropriate restriction enzyme, inserting or adding it into an appropriate site in the bFGF gene using DNA ligase. When any suitable restriction enzyme recognition site is not present in the bFGF gene, restriction enzyme recognition sites may be produced by the above-mentioned site-directed mutagenesis.

For the purpose of obtaining, for example, a mutein lacking bFGF-constituent amino acids, a mutagenic bFGF gene may be produced in 3 different ways. In one of the 3 cases, the amino terminal of bFGF is deleted; in another case, a central region of bFGF is deleted; and in the remaining one case, the carboxyl terminal is deleted.

In the case of deletion of the amino terminal, a codon of the gene which encodes the carboxyl terminal of the amino acid sequence to be deleted is changed to the Met-encoding codon ATG by site-directed mutagenesis, and the codon has an appropriate restriction enzyme recognition site produced in the 5′-terminal side thereof to facilitate its ligation to the promoter.

In the case of deletion of a central region of the amino acid sequence, a unique restriction enzyme recognition site is produced, by site-directed mutagenesis, in each of the 5′-terminal and 3′-terminal sides of the gene which encodes the sequence to be deleted, and the relevant gene is cleaved off from the gene by enzymatic digestion, and this is followed by re-ligation of the remaining 2 gene fragments to construct the desired gene which encodes bFGF lacking the specified amino acids. It is of course necessary not to shift the reading frame due to the digestion with the restriction enzymes.

In the case of deletion of an amino acid sequence in the carboxyl terminal side, a codon of the gene which encodes amino-terminal amino acids of the sequence to be deleted is changed to a stop codon by site-directed mutagenesis.

For the purpose of obtaining a mutein which has had the constituent amino acid cysteine substituted, a mutagenic bFGF gene is produced by eliminating, for example, the Cys-expression codon or inducing site-directed mutagenesis in the Cys-expression codon TGC or TGT using a synthetic nucleotide primer which so changes the codon that it encodes a different amino acid. A primer is hybridized with a sense chain of the FGF gene, for example, to change cysteine (26-position) of human bFGF to serine. As an appropriate nucleotide primer, there may be mentioned, for example, 5'-CGTTCTTGCTGTAGAGCCGCT-3', in which the underlined triplet represents the changed codon.

As an appropriate primer in changing cysteine (70-position) to serine, there may be mentioned 5'-AACGATTAGCGCTCACTC-3', in which the underlined triplet represents the changed codon.

As an appropriate primer for changing cysteine (88-position) to serine, there may be mentioned 5'-GTAACAGACTTAGAAGCTAGT-3', in which the underlined triplet represents the changed codon.

As an appropriate primer for changing cysteine (93-position) to serine, there may be mentioned 5'-TCGAAGAAGAAAGACTCATCC-3', in which the underlined triplet represents the changed codon.

In the case of Cys (26-position), cysteine changes to serine via T→A transpostition of the 1st base; in the case of Cys (70-position), cysteine changes to serine via T→A transposition of the 1st base and T→C transposition of the 2nd base; and in the case of Cys (88-position, 93-position), cysteine changes to serine via G→C transposition of the 2nd base.

It should be noted that, in producing bFGF mutein protein by site-directed mutagenesis, 2 or more variations may be induced in the DNA sequence, that is, the DNA codons corresponding to the amino acids have degenerated.

For the purpose of obtaining a mutein which has had a constituent amino acid other than cysteine substituted by another amino acid, a mutagenic bFGF gene is produced by changing a codon using an oligonucleotide primer in the same manner as with cysteine.

The design of oligonucleotide primer of course varies with which amino acid is to be changed and can be accomplished readily by the skilled artisan.

The primer is hybridized to a single-stranded phage resulting from cloning of a single strand of the bFGF gene, such as M13 [Yanish-Perror, C. Vieira, and J. Messing, Gene, 33, 103-119 (1985); Messing, J., Methods in Enzymology, 101, 20-78 (1983)], fd [R. Herrman et al., Molecular and General Genetics, 177, 231 (1980)] or φ × 174 [M. Smith and S. Gillam, Genetic Engineering, Plenum Press, Vol. 3, pp. 1-32 (1981)]. It is noted that the phage is capable of carrying either the sense chain or antisense chain of the gene. When the phage carries an antisense chain, in addition to discrepancies from the relevant codon determining a triplet which has encoded another amino acid, the primer may have discrepancies due to codon degeneracy from the sense chain region containing the codon to which mutation is to be induced. Similarly, when the phage carries a sense chain, the primer may not be complementary to the sense chain region containing the codon to which mutation is to be induced, as well as appropriate discrepancies from the triplet which pairs to the codon to be deleted. The conditions used for hybridization are described by M. Smith and S. Gillam in the above-mentioned literature. Temperature is normally between about 0°C and 70°C, more generally, between about 10°C and 50°C. After hybridization, the primer is elongated on the phage DNA by reaction with Escherichia coli DNA polymerasa I, T4 DNA polymerase, reverse transcriptase or other appropriate DNA polymerase. The resulting dsDNA is converted to a closed circular dsDNA by treatment with DNA ligase such as T4 DNA ligase. The DNA molecules containing single-strand regions can be decomposed by S1 endonuclease treatment.

The resulting mutational heteroduplex is used to transform an infectable host organism or cell. In the replication of the heteroduplex by the host, progenies are produced from both chains. Following the replication, the mutant gene is isolated from a progeny of the mutant's chain, and is inserted in an appropriate vector, which is then used to transform an appropriate host organism or cell.

The phage DNA carrying the mutational gene is then isolated, and incorporated in a plasmid.

As examples of plasmids in which DNA is incorporated, mention may be made of plasmids deriving from Escherichia coli such as pBR322 [Gene, 2, 95 (1977)], pBR325 [Gene, 4, 121 (1978)], pUC12 [Gene, 19, 259 (1982)] and pUC13 [Gene, 19, 259 (1982)]; and plasmids deriving from Bacillus subtilis such as pUB110 [Biochemical and Biophysical Research Communication, 112, 678 (1983)]; but any other plasmid can also be used, as long as it is replicated and held in the host.

As methods of incorporation in plasmid, there may be mentioned, for example, the method described in Molecular Cloning, T. Maniatis et al., Cold Spring Harbor Laboratory, p. 239 (1982), etc.

The cloned gene is ligated downstream of a promoter in a suitable vehicle (vector) for expression to obtain an expression vector.

Vectors include the above-mentioned plasmids deriving from *Escherichia coli* (e.g., pBR322, pBR325, pUC12 and pUC13), plasmids deriving from *Bacillus subtilis* (e.g., pUB110, pTP5 and pC194), plasmids deriving from yeast (e.g., pSH19 and pSH15), bacteriophages such as λ phage, and animal viruses such as retrovirus and vaccinia virus.

The said gene may have ATG as a translation initiation codon at its 5'-terminal, and may also have TAA, TGA or TAG as a translation termination codon at its 3'-terminal. For the purpose of expression of the said gene, a promoter is ligated upstream thereof. Any promoter can be used for the present invention, as long as it appropriately corresponds to the host used for the gene expression, i.e. it promotes expression in that host.

When the host for transformation is a bacterium of the genus *Escherichia*, *trp* promoter, *lac* promoter, *rec* A promoter, λPL promoter, *lpp* promoter, T7 promoter, etc. are appropriate; when the host is a *bacterium* of the genus Bacillus, SP01 promoter, SP02 promoter, *penP* promoter etc. are appropriate; when the host is a yeast, PHO5 promoter, PGK promoter, GAP promoter, ADH promoter, etc. are appropriate. Specifically, it is preferable that the host be a bacterium of the genus *Escherichia*, and the promoter be *trp* promoter or λPL promoter or T7 promoter.

When the host is an animal cell, appropriate promoters include promoters deriving from SV40 and retrovirus promoters; specifically, promoters deriving from SV40 are preferable.

Using the vector thus constructed, which contains a recombinant DNA having a mutein-encoding base sequence, a transformant is produced.

As hosts, there may be mentioned, for example, bacteria of the genus *Escherichia*, bacteria of the genus *Bacillus*, yeasts, animal cells, insert cells etc.

As examples of said bacteria of the genus *Escherichia*, mention may be made of *Escherichia coli* K12DH1 [Proc. Natl. Acad. Sci. USA, *60*, 160 (1968)], JM103 [Nucleic Acids Research, *9*, 309 (1981)], JA221 [Journal of Molecular Biology, *120*, 517 (1978)], HB101 [Journal of Molecular Biology, *41*, 459 (1969)], C600 [Genetics, *39*, 440 (1954)] and MM294 [Proc. Natl. Acad. Sci. USA, *73*, 4174 (1976)].

As examples of said bacteria of the genus *Bacillus*, mention may be made of *Bacillus subtilis* MI114 [Gene, *24*, 255 (1983)] and 207-21 [Journal of Biochemistry, *95*, 87 (1984)].

As examples of said yeasts, mention may be made of *Saccharomyces cerevisiae* AH22R⁻, NA 87-11A and DKD-5D.

As examples of animal cells, mention may be made of simian cell COS-7, Vero, Chinese hamster ovary cell CHO, mouse L-cell and human FL-cell.

The transformation of the above-mentioned bacteria of the genus *Escherichia* is carried out in accordance with, for example, the methods described in Proc. Natl. Acad. Sci. USA, *69*, 2110 (1972), Gene, *17*, 107 (1982), etc.

The transformation of bacteria of the genus *Bacillus* is carried out in accordance with, for example, the methods described in Molecular and General Genetics, *168*, 111 (1979) etc.

The transformation of yeasts is carried out in accordance with, for example, the method described in Proc. Natl. Acad. Sci. USA, *75*, 1929 (1978).

The transformation of animal cells is carried out in accordance with, for example, the method described in Virology, *52*, 456 (1973).

A transformant harboring a vector containing a recombinant DNA having a mutein-encoding base sequence is thus obtained.

The said transformant is cultured in a medium to allow it to produce such mutein.

In culturing a transformant whose host is a bacterium of the genus *Escherichia* or *Bacillus*, liquid medium is appropriate for the cultivation, in which carbon sources, nitrogen sources, minerals, and other substances essential to the growth of the said transformant are contained. As carbon sources, there may be mentioned, for example, glucose, dextrin, soluble starch, sucrose, etc.; as nitrogen sources, there may be mentioned, for example, inorganic or organic substances such as ammonium salts, nitrates, corn steep liquor, peptone, casein, meat extract, soybean cake and potato extract; as minerals, there may be mentioned, for example, calcium chloride, sodium dihydrogenphosphate and magnesium chloride. Yeasts extracts, vitamins, growth accelerators, etc. may also be added.

It is desirable that the pH of medium be about 6 to 8.

As an example of appropriate medium for the cultivation of bacteria of the genus *Escherichia*, mention may be made of M9 medium containing glucose and casamino acid [Miller, Journal of Experiments in Molecular Genetics, 431-433, Cold Spring Harbor Laboratory, New York (1972)]. Chemicals such as 3β-indolylacrylic acid may be added thereto, when it is necessary to increase promoter efficiency.

When the host is a bacterium of the genus *Escherichia*, cultivation is normally carried out at about 15 to 43° C for about 3 to 24 hours, and, where necessary, aeration and/or agitation are added.

When the host is a bacterium of the genus *Bacillus*, cultivation is normally carried out at about 30 to 40°C for about 6 to 24 hours, and, where necessary, aeration and/or agitation are added.

As a medium for the cultivation of transformants whose host is a yeast, there may be mentioned, for example, Burkholder's minimum medium [Bostian, K. L. et al., Proc. Natl. Acad. Sci. USA, 77, 4505 (1980)]. It is preferable that the pH of the medium be adjusted to about 5 to 8. Cultivation is normally carried out at about 20 to 35°C for about 24 to 72 hours, and, where necessary, aeration and/or agitation are added.

As media for the cultivation of transformants whose host is an animal cell, there may be mentioned, for example, MEM media containing about 5 to 20% fetal bovine serum [Science, 122, 501 (1952)], DMEM medium [Virology, 8, 396 (1959)], RPMI1640 medium [Journal of the American Medical Association, 199, 519 (1967)] and 199 medium [Proceeding of the Society for the Biological Medicine, 73, 1 (1950)]. It is preferable that pH be about 6 to 8. Cultivation is normally carried out at about 30 to 40°C for about 15 to 60 hours, and, where necessary, aeration and/or agitation are added.

The separation and purification of muteins from the above-mentioned culture can be carried out by a number of methods, for example, the following methods can be used.

In extracting mutein from cultured bacterial, fungal or animal cells, there can be employed as appropriate, for example, the method in which bacterial, fungal or animal cells, after cultivation, are collected by a known method, and suspended in a buffer solution containing a protein denaturant such as guanidine hydrochloride to elute the desired protein out of the cells, and the method in which bacterial, fungal or animal cells, after disintegration by French press, ultrasonication, lysozyme treatment and/or freezing-thawing, are subjected to centrifugation to obtain mutein. Specifically, the method using in combination lysozyme treatment and ultrasonication is preferable.

The purification of mutein from the supernatant thus obtained can be carried out by well-known methods of separation and purification in appropriate combination. Examples of such well-known methods of separation and purification include methods based on solubility such as salting-out and solvent precipitation; methods based mainly on the difference in molecular weight such as dialysis, ultrafiltration, gel filtration and SDS-polyacrylamide gel electrophoresis; methods based on the difference in electric charge such as ion-exchange chromatography; methods based on specific affinity such as affinity chromatography; methods based on the difference in hydrophobicity such as reverse phase high performance liquid chromatography; and methods based on the difference in isoelectric point such as isoelectric electrophoresis.

More specifically, by subjecting the above-mentioned supernatant to ion-exchange chromatography using DEAE cellulose etc. as carriers, impurities such as nucleic acids and acid protein can be removed. For example, it is efficient to pass the supernatant through a DEAE cellulose column equilibrated with a nearly neutral buffer solution such as Tris, and collect the fraction not adsorbed to the column. By further subjecting the collected fraction to ion-exchange chromatography using as a carrier CM cellulose or the like, it is possible to adsorb mutein to the carrier, and elute the mutein with a salt solution. These eluates may be lyophilized after dialysis.

Affinity chromatography using heparin-Sepharose can be suitably applied to purifying bFGF mutein in extracts. Thus, for instance, the bFGF mutein protein can be purified by applying the above eluate to a heparin-Sepharose column equilibrated with an almost neutral buffer (e.g. Tris or phosphate buffer), washing the column thoroughly and performing elution by linear gradient constructed with NaCl or the like.

Heparin columns (e.g. Shodex AF-pak HR-894, available from Showa Denko, Japan) developed for high performance liquid chromatography are particularly efficient.

In this case, bFGF mutein can be recovered as homogeneous product in the same manner as in the case of the heparin-Sepharose column mentioned above, namely by applying the sample to a heparin column with an about neutral buffer, washing the column thoroughly and conducting elution on a linear gradient constructed with NaCl, for instance.

The thus-obtained product can be made up into a dry powder form by dialysis and lyophilization. It is desirable to preserve the product with an added carrier (e.g. serum albumin) since the adsorption of the product on the vessel wall can be prevented thereby.

Furthermore, it is preferable to add a slight amount of a reducing agent in the course of purification or preservation, in order to prevent an oxidation of the product.

Examples of the reducing agent, include β-mercaptoethanol, dithiothreitol, glutathione, and so forth.

In this way, substantially pure bFGF mutein protein can be obtained. The substantially pure bFGF mutein protein according to this invention includes products whose bFGF mutein protein content is not less than 95% (w/w) and, more preferably, products whose bFGF mutein content is not less than 98% (w/w).

The mutein thus obtained possesses at least one of the activities of fibroblast growth promoting activity, growth stimulating activity of capillary endothelial cells and angiogenic acitivity, and some of the mutein thus obtained have high stability and are low in toxicity; therefore, it can be used as a healing accelerator for burns, wounds, postoperative tissues, etc., or as a therapeutic drug based on its angiogenic action for thrombosis, arteriosclerosis, etc. It can also be used as a reagent for acceleration of cell cultivation.

Especially, a mutein wherein at least one constituent cysteine is replaced by serine is preferred, because the mutein is remarkably stable.

The mutein according to the present invention, when used as a pharmaceutical, can be safely administered parenterally or orally to warm-blooded animals (e.g., humans, mice, rats, hamsters, rabbits, dogs and cats) directly in a powder form or after preparing as a pharmaceutical composition e.g., injection, tablets, capsules, solutions and ointments) with other pharmacologically allowable carriers, excipients or diluents.

The preparation of injection is carried out in accordance with a routine method using, for example, a physiological saline solution or an aqueous solution containing glucose and other auxiliary agents. Pharmaceutical compositions such as tablets and capsules can also be prepared in accordance with routine methods. When prepared for use as a pharmaceutical, care should be taken that aseptic conditions are used and that the resultant product is sterile, low in pyrogens and endotoxins.

The mutein according to the present invention, when used as any of the above-mentioned pharmaceuticals, is, for example, administered to the above-mentioned warm-blooded animals in an appropriate amount selected in the range of from about 1 ng/kg body weight to 100 μg/kg body weight daily, taking note of the route of administration, symptoms, etc.

The mutein according to the present invention, when used as a reagent for accelerating cell cultivation, is preferably added to the medium so that it is contained therein in an amount of about 0.01 to 10 μg per 1 liter of medium, more preferably about 0.1 to 10 μg per 1 liter of medium.

The mutein of the present invention possesses excellent fibroblast growth promoting activities, vasoendothelial cell growth promoting activities and angiogenic activities, with high stability. It is also very stable in living cells. In addition, replacement of the constituent amino acid cysteine by another amino acid improves stability. The mutein of the present invention can therefore function well as a healing promoter for burns etc., therapeutic drug for thrombosis, arteriosclerosis etc., and cell cultivation promoter.

The abbreviations for bases, amino acids, etc., used in the present specification and the drawings, are based on the abbreviations specified by the IUPAC-IUB Commission on Biochemical Nomenclature or those used commonly in relevant fields, and instances thereof are listed below. When there is a possibility of the presence of an optical isomer in amino acids, the isomer is an L-body unless otherwise specified.

| DNA : | Deoxyribonucleic acid |
|---|---|
| cDNA : | Complementary deoxyribonucleic acid |
| A : | Adenine |
| T : | Thymine |
| G : | Guanine |
| C : | Cytosine |
| RNA : | Ribonucleic acid |
| dATP : | Deoxyadenosine triphosphate |
| dTTP : | Deoxythymidine triphosphate |
| dGTP : | Deoxyguanosine triphosphate |
| dCTP : | Deoxycytidine triphosphate |
| ATP : | Adenosine triphosphate |
| Tdr : | Thymidine |
| EDTA : | Ethylenediaminetetraacetic acid |
| SDS : | Sodium dodecylsulfate |
| Gly : | Glycine |
| Ala : | Alanine |
| Val : | Valine |
| Leu : | Leucine |
| Ile : | Isoleucine |
| Ser : | Serine |
| Thr : | Threonine |
| Cys : | Cysteine |
| Met : | Methionine |
| Glu : | Glutamic acid |
| Asp : | Aspartic acid |
| Lys : | Lysine |
| Arg : | Arginine |
| His : | Histidine |
| Phe : | Phenylalanine |
| Tyr : | Tyrosine |
| Trp : | Tryptophan |
| Pro : | Proline |
| Asn : | Asparagine |
| Gln : | Glutamine |

In the present specification and the drawings, in numbering the human bFGF wherein Met has been added to the N-terminal of the amino acid sequence [III] afore-mentioned is determined as the 1st amino acid, unless otherwise noted.

The following transformants which were produced or used in the Reference Examples or Examples mentioned below were deposited at the Institute for Fermentation, Osaka (IFO), Japan and a- the Fermentation Research Institute, Agency of Industrial Science and Technology, Ministry of International Trade and Industry (FRI), Japan under the accession numbers on the deposit dates shown in Table 1 (The deposit dates are indicated in parentheses.). As to the deposit number of FRI, FERM BP number denotes the number of deposit under the Budapest Treaty; and in case both FERM P number and FERM BP number are described, it shows that the deposit under the number of FERM P has been converted to the deposit under Budapest Treaty and the transformants have been stored at FRI under the number of FERM BP.

Table 1

| Transformants | IFO | FRI | |
|---|---|---|---|
| *E. coli* K12MM294/pTB669 (Reference Example 1) | IFO 14532 (August 11,1986) | FERM P-8918 (August 21,1986) | FERM BP-1281 |
| *E. coli* MM294/pTB762 (Reference Example 3) | IFO 14613 (May 27,1987) | FERM P-9409 (June 11,1987) | FERM BP-1645 |
| *E. coli* DH1/pTB848 (Example 1) | IFO 14674 (November 30,1987) | | FERM BP-1593 (December 7,1987) |
| E. coli MM294/pTB893 (Example 1) | IFO 14772 (August 12,1988) | | FERM BP-2009 (August 22,1988) |
| E. coli MM294/pTB898 (Example 1) | IFO 14773 (August 12,1988) | | FERM BP-2010 (August 22,1988) |
| E. coli MM294/pTB899 (Example 1) | IFO 14774 (August 12,1988) | | FERM BP-2011 (August 22,1988) |
| E. coli MM294/pTB922 (Example 3) | IFO14775 (August 12,1988) | | FERM BP-2012 (August 22,1988) |
| E. coli MM294/pTB923 (Example 4) | IFO14776 (August 12,1988) | | FERM BP-2013 (August 22,1988) |
| E. coli BL21 (DE3) pLysS/pTB956 (Example 6) | IFO 14805 (December 16,1988) | | FERM BP-2202 (December 23,1988) |
| E. coli BL21 (DE3) pLysS/pTB958 (Example 7) | IFO 14806 (December 16,1988) | | FERM BP-2203 (December 23,1988) |

The following hybridomas which were produced in the Example 2(3) mentioned below were deposited at IFO since August 17, 1987 under the accession numbers indicated below.

Mouse HbF 52 cell: IFO 50143
Mouse HbF 78 cell: IFO 50144

Brief Explanation of the Drawings

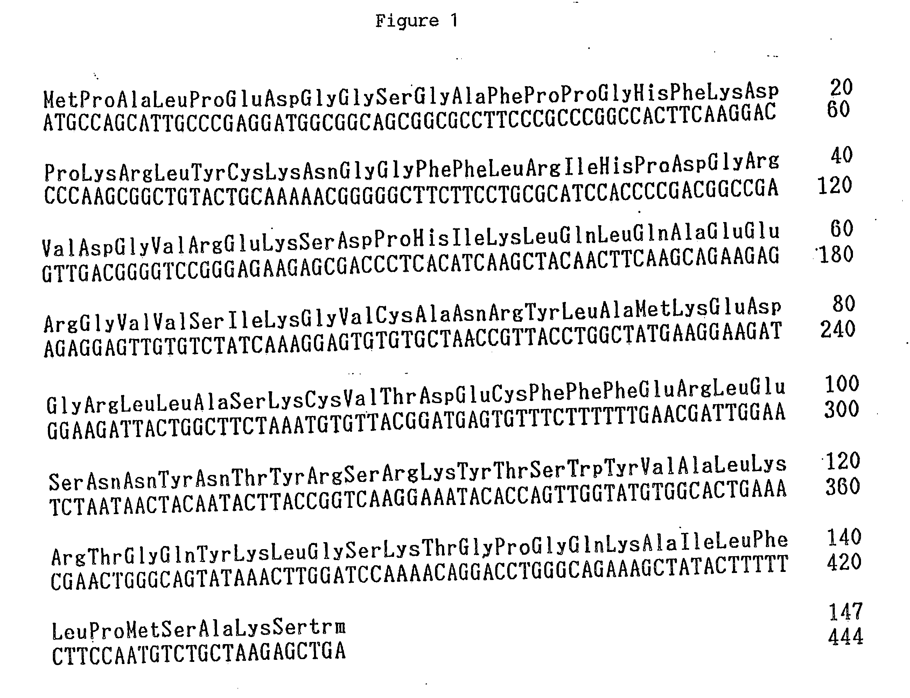

Fig. 1 shows the base sequence which encodes human bFGF and the amino acid sequence deduced therefrom.

Fig. 2 shows the construction scheme of plasmids pTB905 through 911 obtained in Example 1.

Fig. 3 shows the base sequence which encodes rhbFGF mutein CS102, carried by plasmids pTB905 and 893 obtained in Example 1, and the amino acid sequence of rhbFGF mutein C102, encoded thereby.

Fig. 4 shows the base sequence which encodes rhbFGF mutein C105, carried by plasmids pTB906 and 894 obtained in Example 1, and the amino acid sequence of rhbFGF mutein C105, encoded thereby.

Fig. 5 shows the base sequence which encodes rhbFGF mutein C114, carried by plasmids pTB907 and 895 obtained in Example 1, and the amino acid sequence of rhbFGF mutein C114, encoded thereby.

Fig. 6 shows the base sequence which encodes rhbFGF mutein C118, carried by plasmids pTB908 and 896 obtained in Example 1, and the amino acid sequence of rhbFGF mutein C118, encoded thereby.

Fig. 7 shows the base sequence which encodes rhbFGF mutein C123, carried by plasmids pTB909 and 897 obtained in Example 1, and the amino acid sequence of rhbFGF mutein C123, encoded thereby.

Fig. 8 shows the base sequence which encodes rhbFGF mutein C129, carried by plasmids pTB910 and 898 obtained in Example 1, and the amino acid sequence of rhbFGF mutein C129, encoded thereby.

Fig. 9 shows the base sequence which encodes rhbFGF mutein C137, carried by plasmids pTB911 and 899 obtained in Example 1, and the amino acid sequence of rhbFGF mutein C137, encoded thereby.

Fig. 10 shows the construction scheme of plasmid pTB856 obtained in Example 2.

Fig. 11 shows the construction scheme of plasmid pTB861 obtained in Example 1 and plasmid pTB922 obtained in Example 3.

Fig. 12 shows the construction scheme of plasmid pTB923 obtained in Example 4.

EP 0 326 907 A1

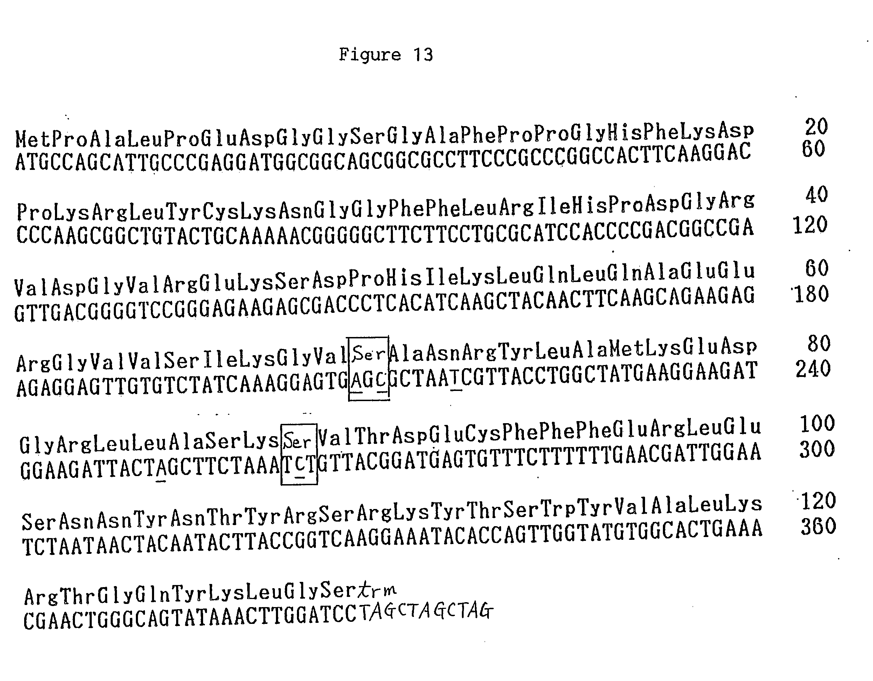

Fig. 13 shows the base sequence which encodes rhbFGF mutein CS23C129, carried by plasmid pTB922 obtained in Example 3, and the amino acid sequence of rhbFGF mutein CS23C129, encoded thereby. The mutated bases are underlined, and each region containing a converted amino acid is surrounded by a square.

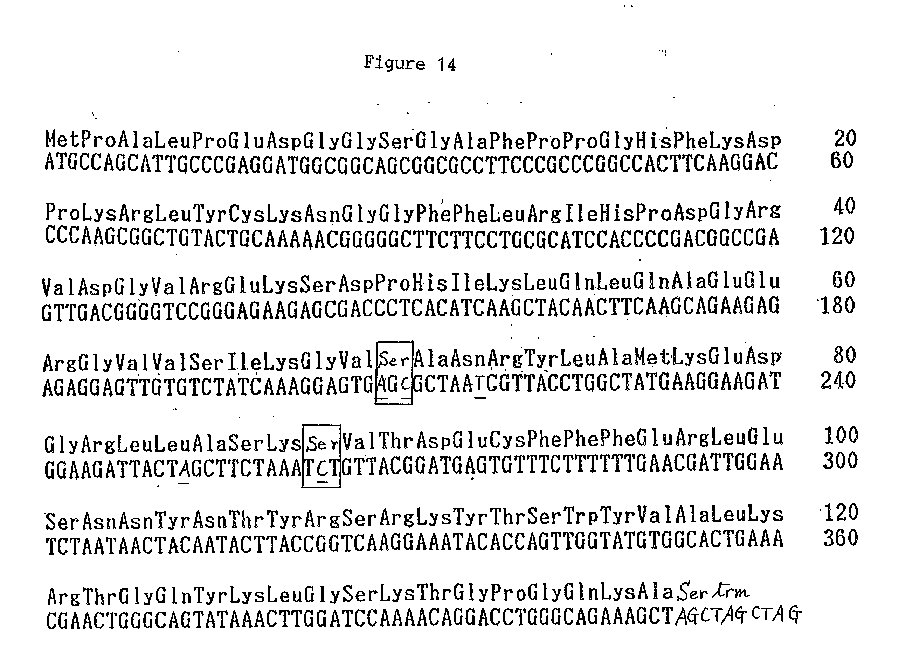

Fig. 14 shows the base sequence which encodes rhbFGF mutein CS23C137, carried by plasmid pTB923 obtained in Example 4, and the amino acid sequence of rhbFGF mutein CS23C137, encoded thereby. The mutated bases are underlined, and each region containing a converted amino acid is surrounded by a square.

Fig. 15 shows the construction scheme of plasmid pTB 956 obtained in Example 6, which encoding rhb FGF mutein C129.

Fig. 16 shows the construction scheme of plasmid pTB 958 obtained in Example 7, which encoding rhb FGF mutein CS23C129.

<u>Reference Example 1</u>

(Production of recombinant DNAS containing mutein-encoding base sequence)

(1) Cloning of M13 vector of human bFGF gene:

The plasmid pTB669 obtained in Example 3 in European Patent Publication No. 237,966 was digested with the restriction enzymes EcoRI and BamHI. The phage vector M13mp8 [J. Messing, Methods in Enzymology, *101*, 20-78 (1983)] replicative-form (RF) DNA was digested with the restriction enzymes EcoRI and BamHI, and was mixed with the human bFGF DNA fragment deriving from the digested pTB669. The mixture was then ligated together by means of T4 DNA ligase; the ligated DNA was transformed into an infectable cell of the strain *Escherichia coli* JM105; the resulting transformant was inoculated onto a plate using Xga1 as an indicator [J. Messing et al., Nucleic Acids Research, *9*, 309-321 (1981)]; the plaque containing the recombinant phage (white plaque) was picked up; the base sequence of the recombinated region was determined by the dideoxynucleotide synthetic chain termination method [J. Messing et al., Nucleic Acids Research, *9*, 309 (1981)], whereby it was confirmed that human bFGF DNA had been accurately inserted.

From this M13-PO clone was purified a single-stranded phage DNA, which was then used as a template for site-directed mutagenesis using a synthetic oligonucleotide.

(2) Site-specific mutagenesis:

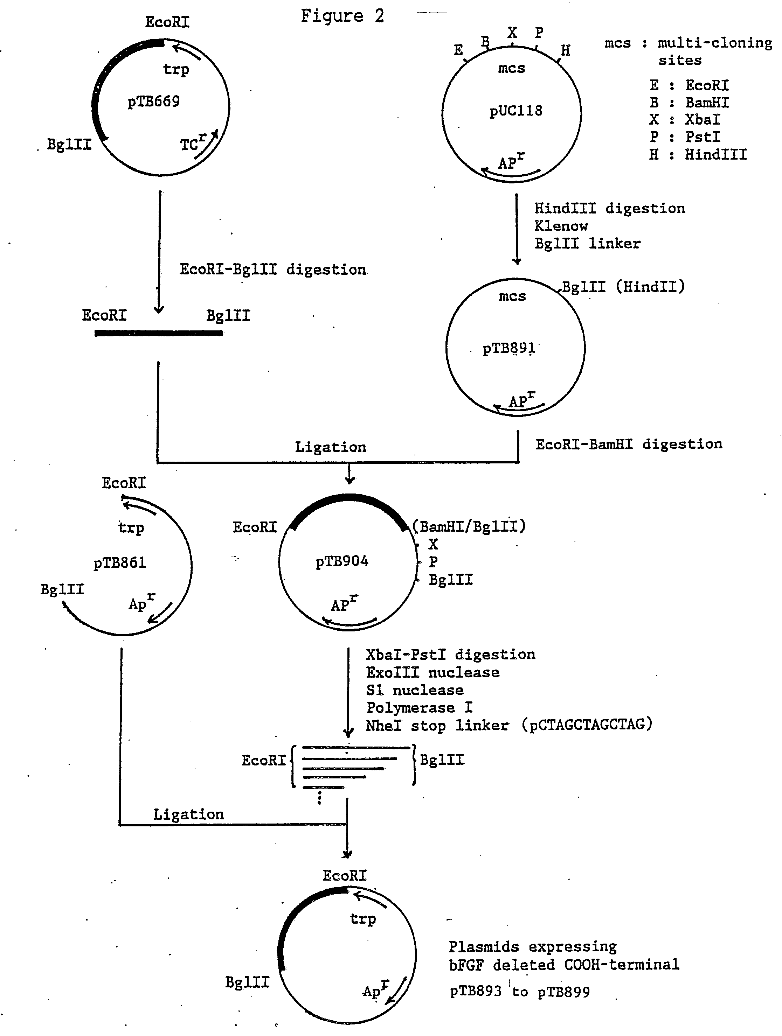

In the presence of 0.1mM adenosine triphosphate (ATP), 50mM Tris (hydroxymethyl)aminomethane hydrochloride (Tris-HCl, pH 8.0), 10mM $MgCl_2$, 5mM dithiothreitol (DTT) and 9 units of T4 kinase, in a total amount of 50 $\mu l$, 40 picomoles of the synthetic oligonucleotide:
5′ > CGT TCT TGC TGT AGA GCC GCT < 3′
[primer for changing *Cys* 26 to *Ser* (the recognition sequence for the restriction enzyme Rsa I disappears.) (see Figure 2)] was treated with T4 kinase at 37°C for 1 hour. This kinase-treated primer (12 picomoles) was heated at 67°C for 5 minutes, and at 42°C for 25 minutes, in 50 $\mu l$ of a mixture containing 50mM NaCl, 1.0mM Tris-HCl (pH 8.0), 10mM $MgCl_2$ and 10mM $\beta$-mercaptoethanol, whereby the primer was hybridized to 5 $\mu g$ of the single-stranded (ss) M13-PO DNA. The annealed mixture was then cooled on ice, and it was added to 50 $\mu l$ of a reaction mixture containing 0.5mM deoxynucleotide triphosphate (dNTP), 80mM Tris-HCl (pH 7.4), 8mM $MgCl_2$, 100mM NaCl, 9 units of DNA plymerase I Klenow fragment, 0.5mM ATP and 2 units of T4 DNA ligase, and reaction was carried out at 37°C for 3 hours, and at 25°C for 2 hours, whereafter the reaction was stopped by adding 2 $\mu l$ of 0.2mM EDTA. The reaction product was used to transform infectable JM 105 cells; the transformant cells thus obtained were allowed to grow overnight, whereafter an ssDNA was isolated from the culture medium supernatant. Using this ssDNA as a template for the 2nd cycle of primer elongation, gel-purified RF-type DNA was transformed into infectable JM 105 cells; the resulting transformant cells were spread over an agar plate, and were cultured overnight to obtain phage plaques.

12

### (3) Site-directed mutagenesis:

The procedure of the above term(2) was repeated but the synthetic oligonucleotide primer used was:
5′ > AAC GAT TAG CGC TCA CTC C <3′
which so changes the cysteine-70-encoding codon that the codon encodes serine. (A recognition sequence of the restriction enzyme HaeII was produced.) (See Figure 2.)

### (4) Site-directed mutagenesis:

The procedure of the above term (2) was repeated but the synthetic oligonucleotide primer used was:
5′ > GTA ACA GAC TTA GAA GCT AGT < 3′ which so changes the cysteine-88-encoding codon that the codon encodes serine. (A recognition sequence for the restriction enzyme AluI was produced.) (See Figure 2.)

### (5) Site-directed mutagenesis:

The procedure of the above term (2) was repeated but the synthetic oligonucleotide primer used was:
5′ > TCG AAG AAG AAA GAC TCA TCC <3′
which so changes the cysteine-93-encoding codon that the codon encodes serine. (A recognition sequence for the restriction enzyme HinfI was produced.) (See Figure 2.)

### (6) Screening and identification of plaques made mutagenic:

Plates containing mutated M13-PO plaques [above term (1)] and 2 plates containing unmutated M13-PO phage plaques were cooled to 4°C, and the plaques from each plate were transferred to 2 round nitrocellulose filters by keeping a dry filter placed on the agar plate for 5 minutes in the case of the lst filter, and for 15 minutes in the case of the 2nd filter. The filters were then kept placed for 5 minutes on thick filter papers immersed in 0.2N NaOH, 1.5M NaCl and 0.2% Triton X-100, after which they were neutralized by keeping them placed for 5 more minutes on filter papers immersed in 0.5M Tris-HCl having a pH-value of 7.5 and 1.5M NaCl. The filters were twice washed on filters immersed in 2 × SSC (standard sodium citrate) in the same manner, and were allowed to dry, and this was followed by drying at 80°C for 2 hours in a vacuum oven. The overlapped filters were subjected to prehybridization at 55°C for 4 hours with 10 mℓ/filter of a DNA hybridization buffer solution (5 × SSC) having a pH-value of 7.0 containing 4 × Denhardt's solution (polyvinylpyrrolidone, Ficoll and bovine serum albumin, 1 × = 0.02%), 0.1% sodium dodecyl sulfate (SDS), 50mM sodium phosphate-buffered solution having a pH-value of 7.0 and 100 μg/mℓ denatured salmon sperm DNA. Hybridization was carried out at 42°C for 24 hours with $10^5$ cpm/mℓ of an oligonucleotide primer. The filters were each washed in a buffer solution for washing containing 0.1% SDS and a reduced amount of SSC at 50°C for 30 minutes. The filters were then first washed with a buffer solution containing 2 × SSC; the control filters, which contained unmutated M13-PO plaques, were examined for radioactivity using a Geiger counter. While stepwise reducing SSC concentration, the control filters were washed until no detectable radioactivity remained on the filters. The minimum of the used SSC concentrations was 0.1 × SSC. The filters were allowed to dry in air, and autoradiographs were taken by 2 to 3 days of exposure at - 70°C. Screening was carried out of 10,000 mutated M13-PO plaques and 100 unmutated control plaques using a kinase-treated oligonucleotide probe. None of the control plaques hybridized to the probe, while 3 to 10 of the mutated M13-PO plaques hybridized to the probe.

One of the mutated M13-PO plaques was taken, and was inoculated to a JM105 culture medium. From the supernatant an ssDNA was prepared, and from the bacterial cell pellets a double-stranded (ds) DNA was prepared. Analyses were made of the base sequences using appropriate oligonucleotide primers and ssDNAs.

As a result, it was respectively confirmed that the TGC (*Cys*-26) codon had been changed to a TCT (*Ser*) codon; the TGT (*Cys*-70) codon, to an AGC (*Ser*) codon; the TGT (*Cys*-88) codon, to a TCT (*Ser*) codon; and the TGT (*Cys*-93) codon, to a TCT (*Ser*) codon.

Of the mutated M13-PO phages, the phage in which the codon *Cys*-26 had become a Ser-encoding codon was named M13-P1; the phage in which the codon *Cys*-70 had become a *Ser*-encoding codon, M13-P2; the phage in which the codon *Cys*-88, M13-P3; and the phage in which the codon *Cys*-93 had become a *Ser*-encoding codon, M13-P4.

## Reference Example 2

(Screening and identification of plaques which were made mutagenic)

Plates containing mutated M13-P2 phage plaques obtained in Reference Example 1 and two plates containing unmutated M13-P2 phage plaques obtained in Reference Example 1 were cooled to 4°C, and the plaque from each plate was transferred to 2 round nitrocellulose filters by keeping a dry filter placed on the agar plate for 5 minutes in the case of the 1st filter, and for 15 minutes in the case of the 2nd filter. The filters were then kept placed for 5 minutes on thick filter papers immersed in 0.2N NaOH, 1.5M NaCl and 0.2% Triton X-100, after which they were neutralized by keeping them placed for 5 more minutes on filter papers immersed in 0.5M Tris-HCl (pH 7.5) and 1.5M NaCl. The filters were twice washed on filters immersed in 2 × SSC (standard sodium citrate) in the same manner, and were allowed to dry, and this was followed by drying at 80°C for 2 hours in a vacuum oven. The overlapped filters were subjected to prehybridization at 55°C for 4 hours with 10 mℓ/filter of a DNA hybridization buffer solution (5 × SSC) having a pH-value of 7.0 containing 4 × Denhardt's solution (polyvinylpyrrolidone, Ficoll and bovine serum albumin, 1 × =0.02%), 0.1% sodium dodecyl sulfate (SDS), 50mM sodium phosphate-buffered solution having a pH-value of 7.0 and 100 μg/mℓ denatured salmon sperm DNA. Hybridization was carried out at 42°C for 24 hours with $10^5$ cpm/mℓ of an oligonucleotide primer. The filters were each washed in a buffer solution for washing containing 0.1% SDS and two times of the amount of SSC at 50°C for 30 minutes. The filters were then first washed with a buffer solution containing 2 × SSC; the control filters, which contained unmutated M13-P2 plaques, were examined for radioactivity using a Geiger counter. While stepwise reducing SSC concentration, the control filters were washed until no detectable radioactivity remained on the filters. The minimum of the SSC concentrations was used 0.1 × SSC. The filters were allowed to dry in air, and radioautographs were taken by 2 to 3 days of exposure at -70°C. Screening was carried out of 10,000 mutated M13-P2 plaques and 100 unmutated control plaques using a kinase-treated oligonucleotide probe. None of the control plaques hybridized to the probe, while 3 to 10 of the mutated M13-P2 plaques hybridized to the probe.

One of the mutated M13-P2 plaques was taken, and was inoculated to a JM105 culture medium. From the resulting supernatant an ssDNA was prepared, and from the bacterial cell pellets a double-stranded (ds) DNA was prepared. Analyses were made of the base sequences using appropriate oligonucleotide primers and ssDNAs.

As a result, it was respectively confirmed that the TGC (*Cys*-26) codon had been changed to a TCT (*Ser*) codon; the TGT (*Cys*-88) codon, to a TCT (*Ser*) codon; and the TGT (*Cys*-93) codon, to a TCT (*Ser*) codon.

Of the mutated M13-P2 phages, the phage in which the codons *Cys*-26 and -70 had become Ser-encoding codons was named M13-P12; the phage in which the codons *Cys*-70 and -88 had become Ser-encoding codons, M13-P23; and the phage in which the codons *Cys*-70 and -93 had become *Ser*-encoding codons, M13-P24.

## Reference Example 3

(Expression in *Escherichia coli* of gene which codes for human bFGF mutein)

(1) Construction of plasmid pTB762 for human bFGF mutein expression:

A replicative form (RF) of M13-P23, obtained in Reference Example 2 above, was cleaved using restriction enzymes EcoRI and PstI to obtain an about 0.5 Kb DNA fragment containing the region which codes for human bFGF mutein.

Separately, the DNA of plasmid ptrp781 [Kurokawa, T. et al., Nucleic Acids Research, *11*, 3077-3085 (1983)], which has trp promoter, was cleaved using ECORI-PstI to separate an about 3.2 Kb DNA fragment containing trp promoter, the tetracycline resistance gene and a plasmid replication initiation site. The above-mentioned 0.5 Kb ECORI-PstI DNA fragment containing the gene region which encodes human bFGF mutein and this 3.2 Kb DNA fragment were ligated together by T4 DNA ligase reaction to construct plasmid pTB762 for human bFGF mutein expression.

Using this plasmid pTB762, *Escherichia coli* MM294 was transformed, whereby the transformant *Escherichia coli* MM294/pTB762 (IFO 14613, FERM BP-1645) was obtained, which harbors plasmid pTB762 containing the gene which codes for rhbFGF mutein CS23, in which Cys at 70- and 88-positions of human bFGF is replaced by Ser.

(2) Preparation of bacterial cell extracts:

The above-mentioned transformant was cultivated in the manner of Example 2 (2) given below to yield a supernatant, which was used as a bacterial cell extract.

(3) Human bFGF activity of bacterial cell extracts:

Mouse BALB/c3T3 cells, in an amount of $2 \times 10^3$ cells for each well, were inoculated to a DMEM medium containing 5% calf serum on a Nunc 96-well microtiter plate (flat base) with each well containing 0.2 m$\ell$ of the medium, and were cultivated. On the following day, the medium was replaced with a DMEM medium containing 0.5% calf serum. After 3 days of cultivation, 10 $\mu\ell$ of a bacterial cell extract, previously serially diluted in 5-fold steps with a DME medium containing 0.5% BSA, was added to each well, and was cultivated. Twenty hours later, 2 $\mu\ell$ of $^3$H-Tdr (5 Ci/mmol, 0.5 mCi/m$\ell$ RCC Amersham) was added to each well. Six hours later, cells were stripped by treatment with a phosphate-buffered solution containing 0.2% trypsin-0.02% EDTA, and the cells were harvested onto a glass filter by means of a Titertech cell harvester, whereafter the amount of $^3$H-Tdr absorbed by the cells was determined using a scintillation counter.

The bacterial cell extracts of *E. coli* DH1/pTB762 exhibited FGF activities.

In the rhbFGF mutein CS23 thus obtained, Cys at 70- and 88-positions of human bFGF was replaced by Ser.

Example 1

(Human bFGF cDNA deletion reaction, construction of mutant, and construction of human bFGF mutein expression plasmid)

From plasmid pTB669, obtained as in Example 3 given in European Patent Publication No. 237,966, a portion corresponding to human bFGF cDNA was isolated using restriction enzymes ECORI and Bgl II, and inserted into plasmid pTB891 [plasmid obtained by converting the Hind III site (multicloning site) of pUC118 (Vieira, J. and Messing J., Methods in Enzymology, in preparation) (purchased by Takara Shuzo Co., Ltd., Japan) to a Bgl II site using Bgl II linker (CAGATCTG) (produced by Takara Shuzo Co., Ltd., Japan)] at the ECORI-BamH I site to construct plasmid pTB904. Plasmid pTB904 thus obtained was cleaved with two restriction enzymes, namely Xba I and Pst I, and subjected to Exo III nuclease reaction and mungbean nuclease reaction using a kilo-sequence deletion kit (produced by Takara Shuzo Co., Ltd.). The plasmid was then ligated with an Nhe I stop linker (produced by New England Biolabs, U.S.A.) and used to transform *Escherichia coli* MV1184 (see Fig. 2).

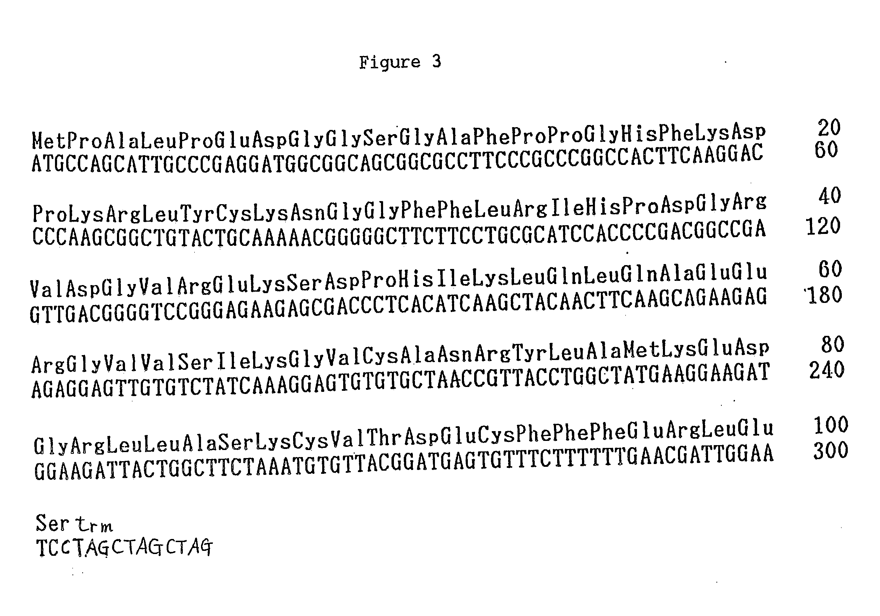

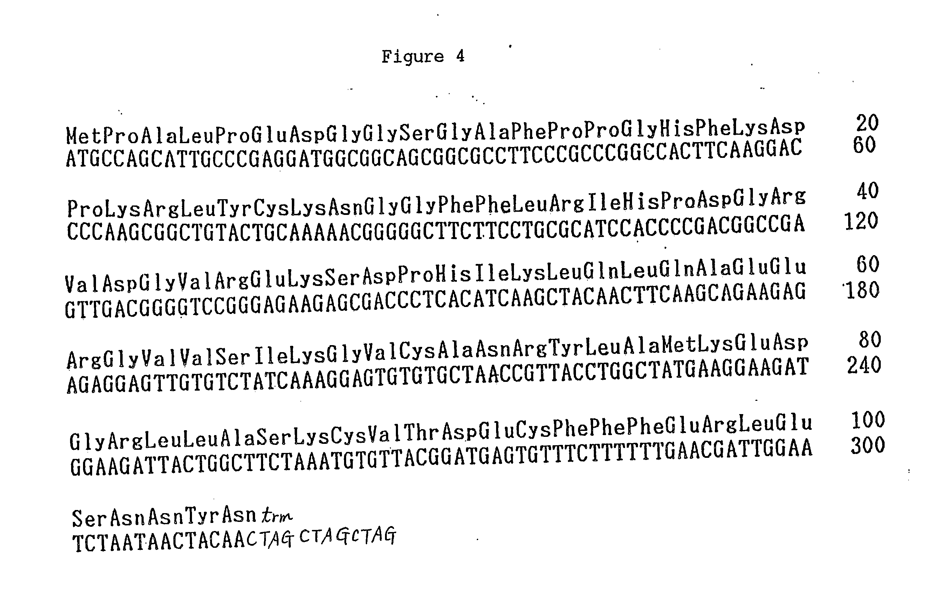

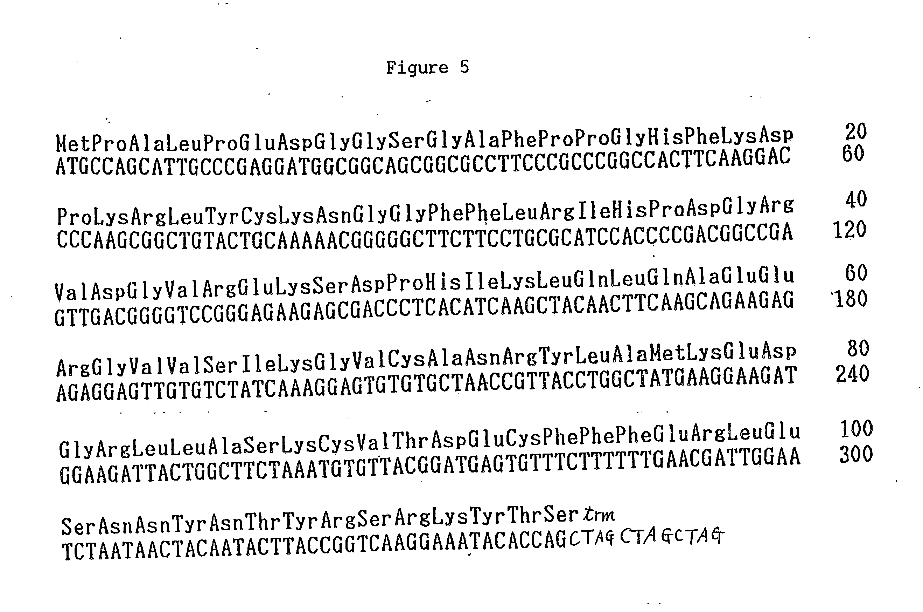

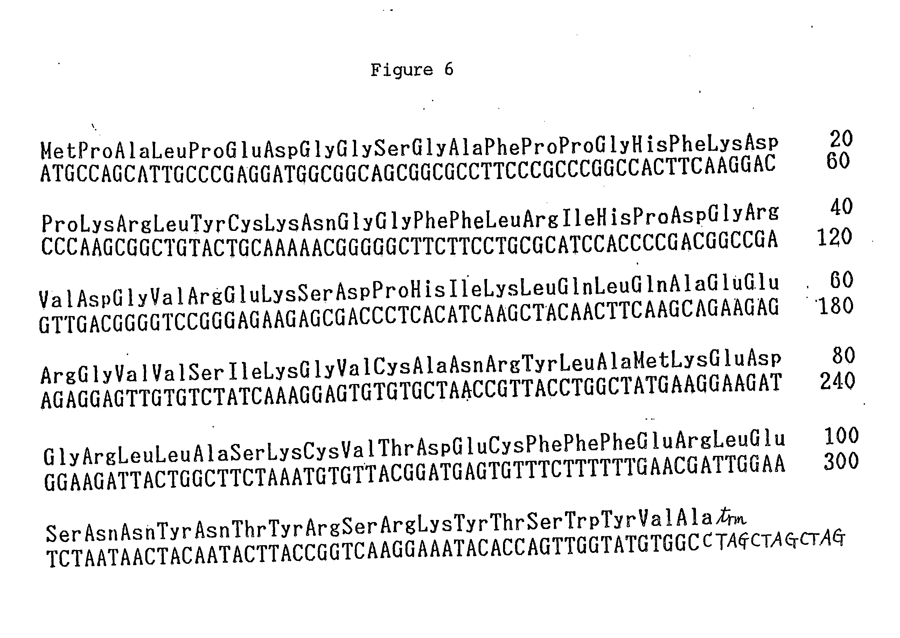

The mutants thus obtained were screened by DNA base sequencing; mutants having any modified portion which codes for the C-terminal of human bFGF were selected. These plasmid mutants were named pTB905 through 911. Their bFGF cDNA portion and bFGF mutein coded for thereby are shown in Fig. 3 through 9, respectively. Plasmid pTB905 encodes rhbFGF mutein C101, which lacks amino acids at 102- to 147-positions of human bFGF (see Fig. 3). Plasmid pTB906 encodes rhbFGF mutein C105, which lacks amino acids at 106- to 147-positions of human bFGF (see Fig. 4). Plasmid pTB907 encodes rhbFGF mutein C114, which lacks amino acids at 115- to 147-positions of human bFGF (see Fig. 5). Plasmid pTB908 encodes rhbFGF mutein C118, which lacks amino acids at 119- to 147 positions of human bFGF (see Fig.

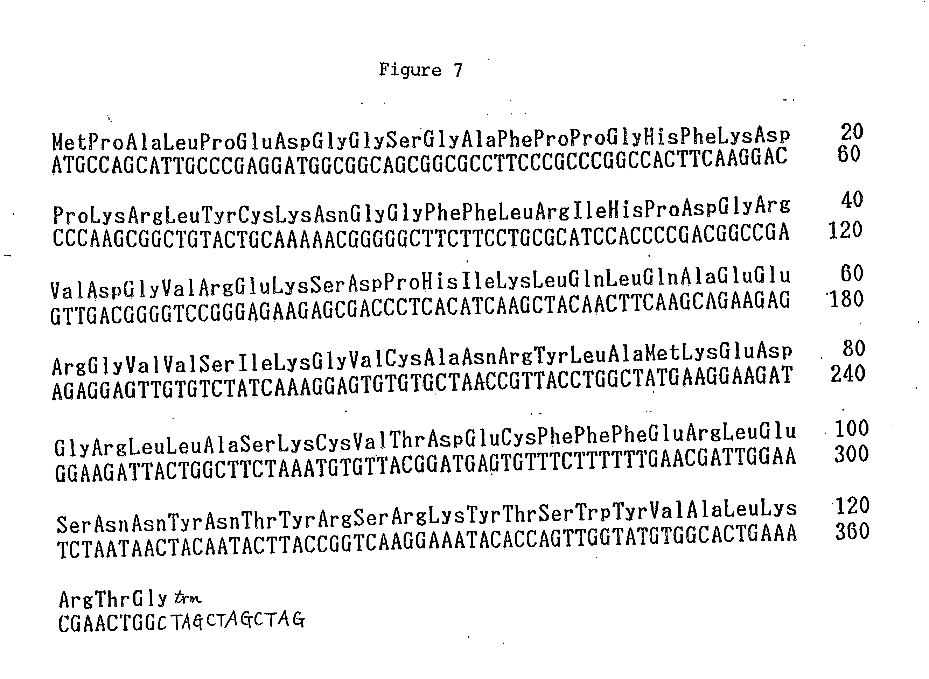

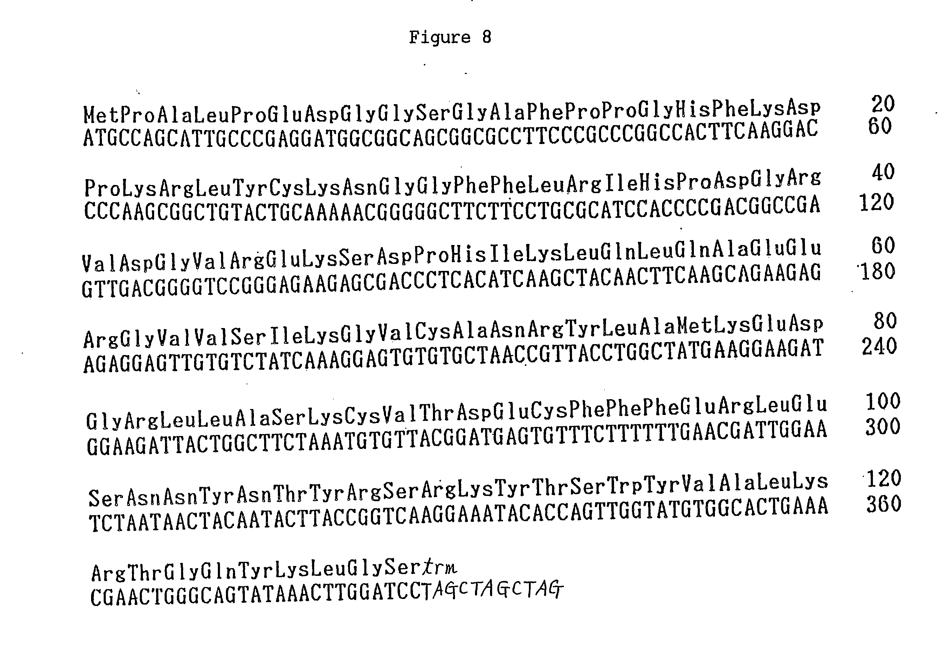

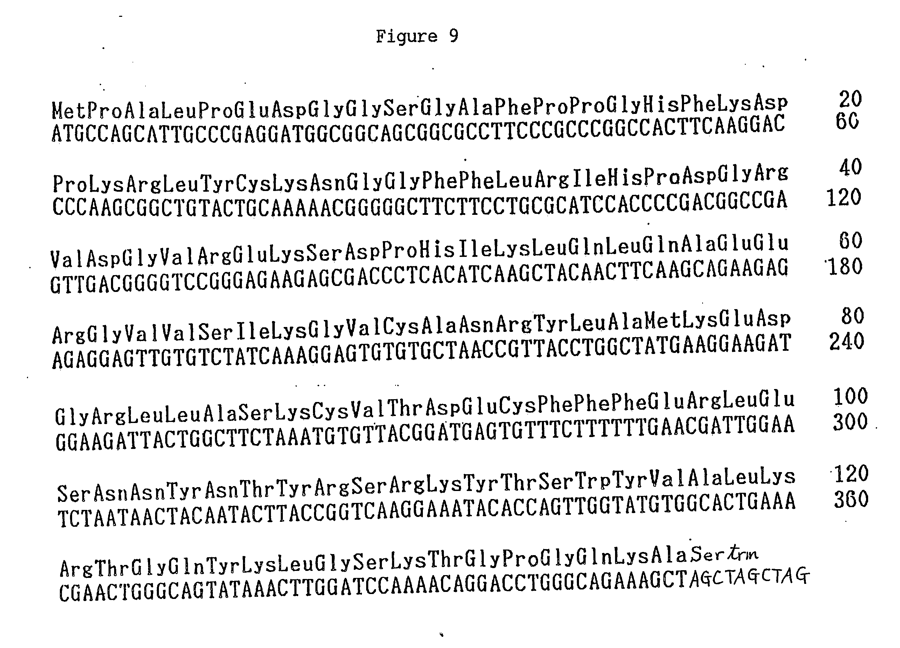

6). Plasmid pTB909 encodes rhbFGF mutein C123, which lacks amino acids at 124- to 147-positions of human bFGF (see Fig. 7). Plasmid pTB910 encodes rhbFGF mutein C129, which lacks amino acids at 130- to 147-positions of human bFGF (see Fig. 8). Plasmid pTB911 encodes rhbFGF mutein C137, which lacks amino acids at 139- to 147-positions of human bFGF and which has serine as the replacement for isoleucine at 138-position (see Fig. 9). Plasmids pTB905 through 911 were each cleaved with ECOR I-Bgl II, and the bFGF cDNA (modified) portion was isolated.

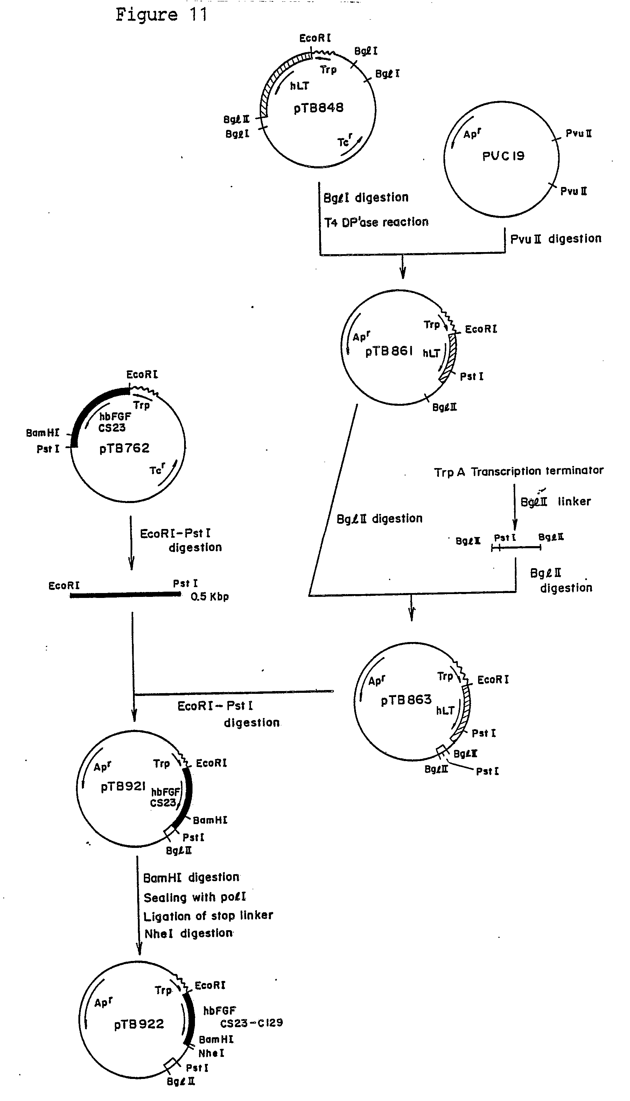

A 1.2 kbp Bgl I DNA fragment containing the region which codes for lymphotoxin was cleaved off from plasmid pTB848, obtained as in Example 6 given in European Patent Publication No. 272, 894. The Bgl I fragment was subjected to T4 DNA polymerase reaction to make both ends blunt, after which it was inserted into plasmid pUC19 [Yanisch-Perron, C. et al., Gene, 33, 103-119 (1985); Messing, J., Methods in Enzymology, 101, 20-78 (1983)] (produced by Pharmacia, Sweden) at the Pvu II site to construct plasmid pTB861 (Fig. 11).

The human bFGF cDNA (modified) portions obtained above were each inserted into the expression vector cleaved with ECOR I-BamH I from plasmid pTB861 obtained above to respectively yield plasmids pTB893, pTB894, pTB895, pTB896, pTB897, pTB898, and pTB899 (Fig. 2). Using these plasmids, E. coli MM294 was transformed to respectively yield the strains: E. coli MM294/pTB893 (IFO 14772, FERM BP-2009), which harbors plasmid pTB893 containing the gene encoding rhbFGF mutein C102, which lacks amino acids at 102- to 147-positions of human bFGF; E. coli MM294/pTB894, which harbors plasmid pTB894 containing the gene encoding rhbFGF mutein C105, which lacks amino acids at 106- to 147-positions of human bFGF; E. coli MM294/pTB895, which harbors plasmid pTB895 containing the gene encoding rhbFGF mutein C114, which lacks amino acids at 115- to 147-positions of human bFGF; E. coli MM294/pTB896, which harbors plasmid pTB896 containing the gene encoding rhbFGF mutein C118, which lacks amino acids at 119- to 147-positions of human bFGF; E. coli MM294/pTB897, which harbors plasmid pTB897 containing the gene encoding rhbFGF mutein C123, which lacks 124- to 147-positions of human bFGF; E. coli MM294/pTB898 (IFO 14773, FERM BP-2010), which harbors plasmid pTB898 containing the gene encoding rhbFGF mutein C129, which lacks amino acids at 130- to 147-positions of human bFGF; and E. coli MM294/pTB899 (IFO 14774, FERM BP-2011), which harbors plasmid pTB899 containing the gene encoding rhbFGF mutein C137, which lacks amino acids at 139- to 146-positions of human bFGF and which has serine as the replacement for isoleucine at 138-position.

Example 2

(Expression in Escherichia coli of gene which encodes human bFGF mutein)

(1) Construction of the plasmid pTB856 for human bFGF mutein expression:

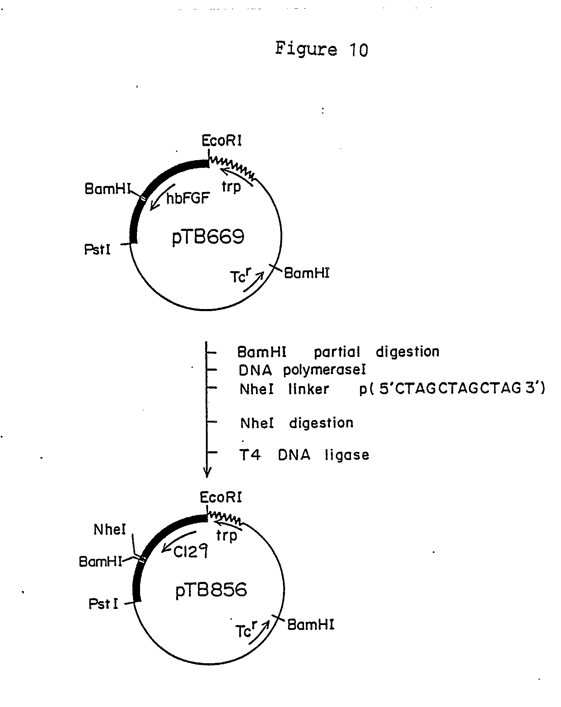

The DNA of the plasmid pTB669 which was obtained in Example 3 of European Patent Publication No. 237,966 was partly cleaved with a restriction enzyme BamHI so as to obtain BamHI recognition site in the bFGF gene. The site was further cleaved with Escherichia coli DNA polymerase I in the presence of dATP, dCTP, dGTP, dTTP to give blunt end. This DNA is ligated with NheI linker p(5′ CTAGCTAGCTAG 3′) under T4 DNA ligase reaction. After treating with the restriction enzyme NheI and ligating the cleaved site under T4 DNA ligase reaction, the plasmid pTB856 for human bFGF mutein expression was constructed (Figure 10).

Using this plasmid pTB856, Escherichia coli MM294 was transformed, whereby the strain Escherichia coli MM294/pTB856 which contains the plasmid pTB856 having the gene coding for rhbFGF mutein C129 which lacks amino acids at 130 at 147 positions of human bFGF.

(2) Preparation of bacterial cell extracts:

The above-mentioned transformants were each cultivated in an M9 medium containing 1% glucose, 0.4% casamino acid and 8 μg/ml tetracycline; when Klett value became about 200, 3β-indolylacrylic acid was added to a concentration of 25 μg/ml, and this was followed by cultivation for 4 more hours. After cultivation, bacterial cells were collected and suspended in a 10% sucrose solution containing a 1/20 amount of 20 mM Tris-HCl, pH 7.6. To this suspension were added phenylmethylsulfonyl fluoride (PMSF) to 1 mM, EDTA to 10 mM, NaCl to 0.1 M, spermidine hydrochloride to 10 mM and lysozyme to 100 μg/ml - (each figure represents final concentration); this mixture was left at 0° C for 45 minutes, after which it was subjected to ultrasonication for 30 seconds. This solution was centrifuged at 18,000 rpm (Sorval centrifuge, SS34 rotor) for 30 minutes to give a supernatant, which was then used as a bacterial cell extract.

(3) Enzyme immunoassay (EIA) (sandwich technique) using the monoclonal antibodies was conducted on the bacterial cell extract obtained in (2) above through procedures (a) through (j) described below. The bacterial cell extract was found to contain FGF. Thus rhbFGF mutein C129 was obtained, which lacks 130-position Lys and amino acids at the succeeding positions.

Said EIA using monoclonal antibody was carried out by the following method:

(a) Immunization:

BALB/c mice (female, 4-week old) had the antigen human bFGF (as obtained in Example 3 of European Patent Publication No. 237,966 Example 3 of European Patent Publication No. 237,966) in solution in 0.4 ml of Freund's complete adjuvant (Difco Laboratories, USA) injected intraperitoneally. Three weeks later, 10 μg of the antigen hbFGF in solution in 0.4 ml of Freund's incomplete adjuvant was intraperitoneally administered. 3 weeks later, the same additional immunization was carried out, and, two weeks later, 10 μg of human bFGF in solution in physiological saline was intraperitoneally inoculated.

(b) Cell fusion:

From the immunized mice mentioned in said (a), the spleen was excised 4 days after final antigen challenge to thereby obtain cells to be used for cell fusion. These cells were suspended in a medium prepared by mixing together Isokov medium and Ham F-12 medium in a ratio of 1:1 (hereinafter referred to. as IH medium).

The mouse myeloma cell P3-X63-Ag 8Ul was subcultured in RPMI 1640 medium containing 10% fetal bovine serum under an atmosphere of 5% carbon dioxide and 95% air.

Cell fusion was conducted in accordance with the method established by Köhler and Milstein [Köhler, G. and Milstein, C.: Nature, 256,495 (1975)].

$2.9 \times 10^7$ cells of the above myeloma cell line and $1.5 \times 10^8$ immunized lymphocytes obtained by the above-mentioned method were mixed together and centrifuged, and 45% polyethylene glycol 6000 (hereinafter referred to as PEG 6000) in solution in 0.3 ml of IH medium was dropwise added. The PEG 6000 solution was preheated to 37° C, and was gradually added. Five minutes later, the 37° C-preheated IH medium was added at a rate of 0.5 ml per minute to make 10 ml. The solution was then centrifuged at room temperature at 600 rpm for 15 minutes, and the supernatant was removed. This cell precipitate was suspended in 200 ml of IH medium containing 20% calf serum, and this suspension was transferred to a 24-well microplate (Linbro) in an amount of 2 ml per well. One day later, IH medium (containing 20% calf serum) supplemented with HAT ($1 \times 10^{-4}$ M hipoxanthine, $4 \times 10^7$ M aminopterin, $1.6 \times 10^{-4}$ M thymidine) (hereinafter referred to as HAT medium) was added to the microplate in an amount of 1 ml per well, and, a further three days later, one half amount of the medium was replaced with HAT medium. The cells thus grown are hybrid cells.

(c) Search for antibody-producing cells:

Previously, the hybrid cell culture supernatant was added in an amount of 100 Hl per well to a 96-well microtiter plate made of polystyrene which had had human bFGF immobilized thereto, and incubation was carried out at room temperature for 2 hours. The incubation supernatant was removed, and, after washing, the horse radish peroxidase (HRP)-labeled anti-mouse IgG goat antibody (Miles Laboratories, U.S.A.) was added as the secondary antibody, and incubation was carried out at room temperature for 2 hours. The secondary antibody was removed, and, after thoroughly washing the wells, coloring reaction was carried out in the presence of added reaction substrate (EIA method). By this method potent valency was observed in 3 wells.

(d) Cloning of hybrid cells:

Cells in each of these three cells were sown to 0.5 cell per well to a 96-well microtiter plate which had had $10^4$ cells/well mouse thymocytes as vegetative cells sown thereon, and cloning was carried out. As a result, three clones, namely the mouse hybridoma HbF52 (IFO 50143) and the mouse hybridoma HbF78 (IFO 50144) were obtained.

(e) Ascitization of hybrid cells:

2 × 106 cells of each of the mouse hybridoma HbF52 or the mouse hybridoma HbF78 was inoculated to mice which had had mineral oil previously administered intraperitoneally. Ten days later, ascites in an amount of 2 ml was collected from each mouse, whereby the monoclonal antibody MoAb52 and the monoclonal antibody MoAb78 were respectively obtained.

(f) Thus obtained monoclonal antibody MoAb 78 was subjected to purification from ascites fluid in accordance with the method of Stachelin et al. The Journal of Biological Chemistry, 256, 9750 (1981). Thus obtained antibody was concentrated to more than 2 mg/ml, and subjected to dialysis in 0.2M sodium phosphate buffer (pH 7.0M. To thus obtained 1.4 ml solution of MoAb 78 (concentration 2.8 mg/ml), 50 µl of S-acetylmercaptosuccinic anhydride (Aldrich Co., U.S.A) dissolved in N, N´-dimethylformamide was added so as to reach the concentration of 11.5 mg/ml. The air in the reaction vessel is replaced by nitrogen gas. The vessel was sealed, and subjected to stirring so as to cause the reaction of introducing SH group. The unreacted S-acetylmercaptosuccinic acid anhydride was inactivated by the treatment for 10 minutes with 130 µl of 0.2M Tris HCl (pH 7.0), 13 µl of 0.2M EDTA and 130 µl of 2M hydroxyamine (pH 7.0). The MoAb 78 was recovered by gel filtration using a column packed with Sephadex G-25 (diameter 1 cm × 80 cm, pharmacia, Sweden) (flow rate: 20 ml/hour).

(g) 10 mg of horse radish peroxidase (HRP, Behringer Manheim, Grade I, West Germany) was dissolved in 1.4 ml of 0.1M phosphate buffer (pH 6.8). On the other hand, 14 mg of N-hydroxysuccinimide ester of N-(4-corboxy cyclohexyl methyl) maleimide was dissolved in 335 µl of DMF, and 100 µl of thus obtained solution was added to the HRP solution above mentioned. The air in the reaction vessel was replaced by nitrogen gas, and the vessel was sealed. After 1 hour reaction at room temperature, proteins of the portion of HRP introduced with maleimide group were recovered by gel filtration using a colomn packed with Sephadex G-25 as in the above (f).

(h) 6 ml of the portion of the monoclonal antibody MoAb 78 introduced with SH group obtained in the above (f) and 2 ml of the portion of HRP introduced with maleimide group obtained in the above (g) were mixed, and the mixture was concentrated to 1 ml using collodion bag (Sartorius, West Germany) under reduced pressure at 4°C for 20 hours. After the reaction, the unreacted HRP introduced with SH group was removed with the use of Ultrogel AcA44 (LKB Co., diameter 1 cm × 80 cm, Sweden) column (flow rate: 10 ml/hour). In the eluates, the fraction containing 2.4 HRP/antibody has the most high HRP number per antibody molecule. The product thus obtained was employed in the EIA in the following item (i).

(i) The monooclonal antibody MoAb 52 obtained in the above (e) was purified by the manner described in the above (f). The monoclonal antibody MoAb 52 was diluted with PBS so as to be 10 µg/ml or 20 µg/ml, and the diluent was poured into Immunoplate (Nunc. Denmark) so as to be 100 µl/well. The plate was kept standing at 4°C overnight to adsorbe the monoclonal antibody MoAb 52 to the plate. After removing the antibody which is not adsorbed, the plate was washed with PBS thrice, PBS containing 0.01% merthiolate and 1% bovine serum albumin (BSA) was added to the plate at 200 µl/well, and the plate was kept standing at 4°C overnight.

(j)The cell extract containing bFGF mutein C129 obtained in the above (2) was diluted with PBS containing 0.1% BSA. From the plate obtained in the above (i), BSA solution was removed, the plate was washed with PBS four times, and the diluted bFGF mutein C129 was added to the plate so as to be 100 µl/well to adsorb to the plate at 4°C overnight. The unreacted mutein C129 was removed, and the plate was washed with PBS four times. The monoclonal antibody conjugated with HRP obtained in the above (h) was diluted with PBS containing 0.1% BSA to 1/300, and the diluent was added to the plate so as to be 100 µl/well. The reaction was carried out for 4 hours at room temperature. After removing the antibody, the plate was washed with PBS for 6 times, substrate for oxidase (Bio Rad Co. U.S.A) was added to the plate so as to be 100 µl/well. Quantification was accomplished by absorbance measurements at 415 nm, and it was confirmed that a small amount of the mutein C129 was produced.

18

## Example 3

(1) Construction of the expression plasmid

Trp A transcription terminator comprising 28 base pairs (produced by Pharmacia, Sweden)

$$\left(\begin{array}{l} \text{AGCCCGCCTAATGAGCGGGCTTTTTTTT} \\ \text{TCGGGCGGATTACTCGCCCGAAAAAAAA} \end{array}\right) \text{,after being ligated with}$$

$$\text{BgI II linker} \quad \left(\begin{array}{l} \text{GCAGATCTGC} \\ \text{CGTCTAGACG} \end{array}\right) \text{by T4 DNA Ligase, was digested with}$$

Bgl II and inserted into plasmid pTB861 as produced in Example 1 at the Bgl II site using T4 DNA ligase to construct plasmid pTB863. Separately, an ECORI-PstI DNA fragment which codes for rhbFGF mutein CS23 was cleaved off from plasmid pTB762, obtained in Reference Example 3, and was inserted into plasmid pTB863 at the ECORI-Pst I site to construct plasmid pTB921. This plasmid pTB921 was cleaved with BamH I and subjected to *E. coli* DNA polymerase I (Klenow enzyme) reaction to duplicate the single-strand portion. This DNA fragment was then ligated with translation termination linker

$$\left(\begin{array}{l} \text{CTAGCTACCTAG} \\ \text{GATCGATGGATC} \end{array}\right) \text{using T4 DNA ligase,}$$

after which it was trimmed with Nhe I and cyclized using T4 DNA ligase to construct plasmid pTB922 (see Fig. 11). This plasmid was used to transform *E. coli* MM294 to give *E. coli* MM294/pT922 (IFO 14775, FERM BP-2012). This expression plasmid pTB922 expresses hbFGF mutein CS23C129, which is of rhbFGF mutein CS23 type and which lacks amino acid residues at 130- to 147-positions from the C-terminal.

(2) Preparation of cell extract

The above transformants was cultivated in M9 medium containing 1% glucose, 0.4% casamino acids and 8 μg/ml tetracycline. When the Klett value was about 200, 3-β-indolylacrylic acid was added to 25 μg/ml, and the cultivation was continued for further 4 hours. Thereafter, cells were harvested and suspended in one twentieth volume of 10% sucrose solution in 20 mM Tris-HCl, pH 7.6. To this suspension were added phenylmethylsulfonyl fluoride (PMSF) to 1 mM (final concentration), EDTA to 10 mM, NaCl to 0.1 M, spermidine hydrochloride to 10 mM and lysozyme to 100 μg/ml. After allowing to stand at 0°C for 45 minutes, the whole mixture was sonicated for 30 seconds. The sonication product was centrifuged at 18,000 rpm (Servall centrifuge, SS 34 roter, U.S.A.) for 30 minutes to give a supernatant, which was used as the cell extract.

(3) rhbFGF mutein detected in cell extract

rhbFGF mutein CS23C129 in the cell extract was detected by immunological method using monoclonal antibodies against humman bFGF in a manner of Example 2.

## Example 4

(1) Construction of expression plasmid pTB923

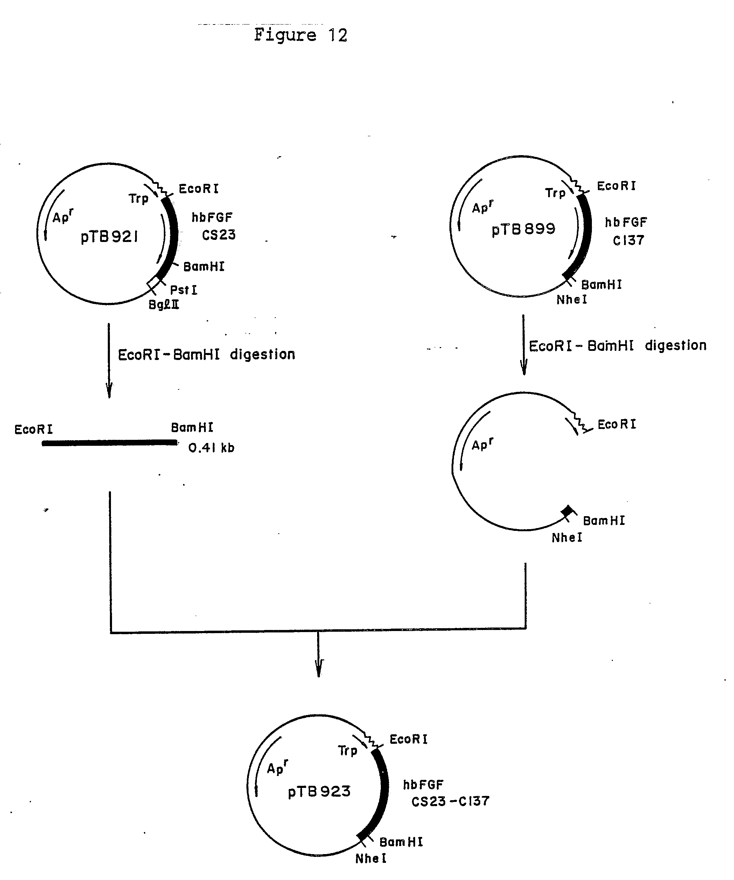

Plasmid pTB921 as obtained in Example 3 was digested with ECOR I and BamH I to give a 0.41 kb ECOR I-BamH I fragment, which was then inserted into plasmid pTB899, which expresses rhbFGF mutein C137, which lacks amino acid residues at 139- through 147-positions in the C-terminal and which has serine as the replacement for isoleucine at 138-position, at the ECOR I-BamH I site to construct plasmid pTB923 (see Fig. 12). Using this plasmid, E. coli MM294 was transformed to yield E. coli MM294/pTB923 (IFO 14776, FERM BP-2013).

Plasmid pTB923 expresses hbFGF mutein CS23C137, which is of mutein CS23 type and which lacks amino acid residues at 139- through 147-positions at the C-terminal and which has serine as the replacement for isoleucine at 138-position.

(2) Preparation of cell extract The above transformants was cultivated in M9 medium containing 1% glucose, 0.4% casamino acids and 8 μg/mℓ tetracycline. When the Klett value was about 200, 3-β-indolylacrylic acid was added to 25 μg/mℓ, and the cultivation was continued for further 4 hours. Thereafter, cells were harvested and suspended in one twentieth volume of 10% sucrose solution in 20 mM Tris-HCℓ, pH 7.6. To this suspension were added phenylmethylsulfonyl fluoride (PMSF) to 1 mM (final concentration), EDTA to 10 mM, NaCℓ to 0.1 M, spermidine hydrochloride to 10 mM and lysozyme to 100 μg/mℓ. After allowing to stand at 0°C for 45 minutes, the whole mixture was sonicated for 30 seconds. The sonication product was centrifuged at 18,000 rpm (Servall centrifuge, SS 34 roter, U.S.A.) for 30 minutes to give a supernatant, which was used as the cell extract.

(3) rhbFGF mutein detected in cell extract

rhbFGF mutein CS23C129 in the cell extract was detected by immunological method using monoclonal antibodies against humman bFGF in a manner of Example 2.

Example 5

(Bioactivities of the rhbFGF mutein C129)

FGF activity of the bacterial cell extract of E. coli MM294/pTB856 obtained in Example 2 (2) was determined by the method described in Reference Example 3 (3) The addition of the bacterial cell extract evidently promoted ³H-thymidine uptake of BALB/c3T3 cells in a stationary state.

The preparation purified from the bacterial cell extract by heparin affinity chromatography was also assayed for FGF activity in the manner as mentioned above; the rhbFGF mutein C129 exhibited an activity of 0.02 to 0.1, relative to that of FGF, taken as 1.

The preparation as mentioned above was also examined for effect on the proliferation of BALB/c3T3 cells. BALB/c3T3 cells were seeded to a 24-well plate at $10^4$ cells/well, each well holding a DMEM medium containing 5% FCS. The next day the medium was replaced by a DMEM medium containing 0.6% FCS with the above-mentioned preparation added simultaneously. After a 3-day cultivation, cells were counted using a Coulter counter. The results are shown in Table 2 shown below.

Table 2

| | rhb FGF mutein C129 | | | | |
|---|---|---|---|---|---|
| | 0 | 0.2 | 1 | 5 | 25 |
| Cells($10^4$) | 3.30 | 5.08 | 6.23 | 6.77 | 8.17 |

As seen in the Table 2, rhbFGF mutein C129, at concentrations of 5 to 25 ng/mℓ, increased the cell count to 2 to 2.5 times the original levels. In addition, although bFGF causes noticeable morphological changes in BALB/C3T3 cells at concentrations of 0.5 to 1.0 ng/mℓ or more, no morphological changes occurred in BALB/c3T3 cells even when rhbFGF mutein C129 was added at 25 ng/mℓ.

## Example 6

(Production of human bFGF mutein C129 using a T7 promoter)

(1) The DNA of the plasmid pTB898 obtained in Example 1, which carries the gene which codes for rhbFGF mutein C129, was digested with the restriction enzyme ECOR I; the single-stranded portion was digested by mung bean nuclease treatment to make both ends blunt, after which it was cleaved with the restriction enzyme Bgl II to give a fragment which codes for rhbFGF mutein C129.

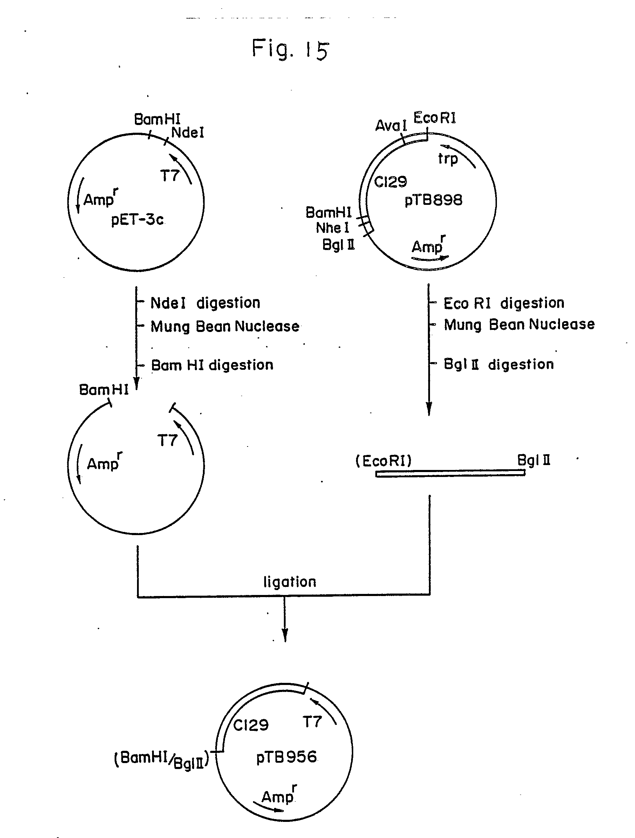

Separately, the DNA of the expression vector pET-3c [described in Alan H. Rosenberg et al., Gene, 56 (1987), pp. 125-135], which carried T7 promoter, was digested with the restriction enzyme NdeI; the single-stranded portion was digested by mung bean nuclease treatment to make both ends blunt, after which it was further digested with BamH I. The above-mentioned fragment was inserted into this portion in the presence of T4 DNA ligase to construct the C129 expression plasmid pTB956 (Fig. 15).

This plasmid was used to transform Escherichia coli BL21 (DE3) pLysS [F. William Studler and Barbara A. Moffatt, Journal of Molecular Biology, 189 (1986), pp. 113-130] to give the transformant E. coli BL21 (DE3) pLysS/pTB956 (IFO 14805, FERM BP-2202), which carried the plasmid pTB956 containing the gene which codes for human bFGF mutein C129.

(2) Preparation of bacterial cell extract

The above-mentioned transformant was cultivated in an LB medium containing 10 μg/ml chloramphenicol and 35 μg/ml ampicillin. When Klett value became 180, isopropyl-β-D-thiogalactoside (IPTG) was added to 0.5 mM; cultivation was continued for 3 more hours. After cultivation, cells were harvested and suspended in a 10% sucrose solution containing 20 mM Tris HCl, pH 7.6, which was 1/20th the volume of the cultured broth. To this suspension were added phenylmethylsulfonyl fluoride (PMSF) to 1 mM, EDTA to 10 mM, NaCl to 0.1 M, spermidine hydrochloride to 10 mM, and lysozyme to 100 μg/ml (every figure shows the final concentration); this mixture was left at 0°C for 45 minutes, after which it was ultrasonicated for 30 seconds. This solution was then centrifuged at 18,000 rpm (Sorval centrifuge, SS-34 rotor) for 30 minutes to give a supernatant, which was used as a bacterial cell extract.

(3) The bacterial cell extract obtained in (2) above was subjected to enzyme immunoassay (EIA) (sandwich technique) using monoclonal antibodies in the steps (a) through (j) of Example 2. The bacterial cell extract was found to contain FGF. The rhbFGF mutein C129, which lacks the Lys130 and succeeding amino acids, was thus obtained.

## Example 7

(Production of human bFGF mutein CS23C129 using a T7 promoter)

(1) The DNA of the plasmid pTB762 obtained in Reference Example 3, which expresses rhbFGF mutein CS23, was digested with the restriction enzymes Ava I and BamH I to give a region domain containing the Ser70 and Ser88 of CS23.

The DNA of the plasmid pTB956 obtained in Example 6 above was digested with the restriction enzymes Ava I and Sal I to yield a fragment containing a T7 promoter. Separately, the DNA of pTB956 was digested with the restriction enzymes Sal I and BamH I to give a fragment containing the ampicillin resistance gene.

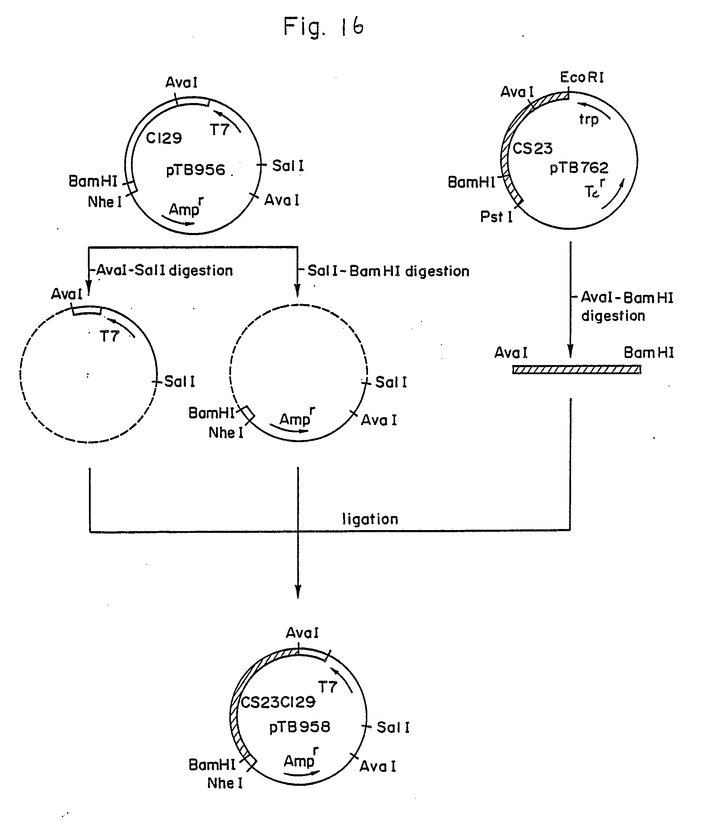

The three DNA fragments thus obtained were ligated together by T4 DNA ligase to construct the rhbFGF mutein CS23C129 expression plasmid pTB958 (Fig. 16).

This plasmid was used to transform Escherichia coli BL21 (DE3) pLysS [F. William Studler and Barbara A. Moffatt, Journal of Molecular Biology, 189 (1986), pp. 113-130] to give the transformant E. coli BL21 (DE3) pLysS/pTB958 (IFO 14806, FERM BP-2203), which carries the plasmid pTB958 containing the gene which codes for rhbFGF mutein CS23C129.

(2) Preparation of bacterial cell extract

The above-mentioned transformant was cultivated in an LB medium containing 10 μg/mℓ chloramphenicol and 35 μg/mℓ ampicillin. When Klett value became 180, isopropyl-β-D-thiogalactoside (IPTG) was added to 0.5 mM; cultivation was continued for 3 more hours. After cultivation, cells were harvested and suspended an a 1/20 amount ofa 10% sucrose solution containing 20 mM Tris HCl, pH 7.6. To this suspension were added phenylmethylsulfonyl fluoride (PMSF) to 1 mM, EDTA to 10 mM, NaCl to 0.1 M, spermidine hydrochloride to 10 mM, and lysozyme to 100 μg/mℓ (every figure shows the final concentration); this mixture was left at 0°C for 45 minutes, after which it was ultrasonicated for 30 seconds. This solution was then centrifuged at 18,000 rpm (Sorval centrifuge, SS-34 rotor) for 30 minutes to give a supernatant, which was used as a bacterial cell extract.

(3) The bacterial cell extract obtained in (2) above was subjected to enzyme immunoassay (EIA) (sandwich technique) using monoclonal antibodies in the steps (a) through of Example 2. The bacterial cell extract was found to contain FGF. The rhbFGF mutein C23C129, which lacks the Lys130 and succeeding amino acids, was thus obtained.

## Claims

1. A mutein of basic fibroblast growth factor (bFGF), which lacks 7 to 46 amino acids of the constituent amino acids of bFGF from carboxyl terminal.

2. A mutein as claimed in Claim 1, wherein further at least one constituent amino acid is replaced by other amino acid or at least one amino acid is added to the amino terminal.

3. A mutein as claimed in Claim 1, wherein the bFGF includes the amino acid sequence:
Phe-Phe-Leu-Arg-Ile-His-Pro-Asp-Gly-Arg-Val-Asp-Gly-Val-Arg-Glu-Lys-Ser-Asp-Pro
in its molecule.

4. A mutein as claimed in Claim 1, wherein the bFGF is human bFGF.

5. A mutein as claimed in Claim 2, wherein the constituent amino acid which is to be replaced is cysteine.

6. A mutein as claimed in Claim 2, wherein at least one cysteine is replaced by a neutral amino acid.

7. A mutein as claimed in Claim 6, wherein the neutral amino acid is serine or threonine.

8. A mutein as claimed in Claim 1, wherein the mutein is rhbFGF mutein CS23C129, in which amino acid residues at 130-(Lys) through 147-(Ser) positions of human bFGF are lacking and Cys at 70- and 88-positions are replaced by Ser.

9. A mutein as claimed in Claim 1, wherein the mutein is rhbFGF mutein CS23C137, in which amino acid residues at 138-(Ile) through 147-(Ser) positions of human bFGF are lacking, Cys at 70- and 88-positions are replaced by Ser, and Ile at 138-position is replcaed by Ser.

10. A recombinant DNA having a base sequence which codes for a mutein of basic fibrobalst growth factor (bFGF), the mutein lacking 7 to 46 amino acids of the constituent amino acids of bFGF from carboxyl terminal.

11. A recombinant DNA as claimed in Claim 10, wherein further at least one constituent amino acid is replaced by other amino acid or at least one amino acid is added to the amino terminal.

12. A recombinant DNA as claimed in Claim 11, wherein at least one cysteine is replaced by a neutral amino acid.

13. A transformant harboring a vector containing a recombinant DNA having a base sequence which codes for a mutein of basic fibroblast growth factor (bFGF), the mutein lacking 7 to 46 amino acids of the constituent amino acids of bFGF from carboxyl terminal.

14. A transformant as claimed in Claim 13, wherein further at least one constituent amino acid is replaced by other amino acid or at least one amino acid is added to the amino terminal.

15. A transformant as claimed in Claim 14, wherein at least one cysteine is replaced by a neutral amino acid.

16. A method for producing mutein of basic fibroblast growth factor (bFGF), the mutein lacking 7 to 46 amino acids of the constituent amino acids of bFGF from carboxyl terminal, which comprises cultivating a transformant harboring a vector containing a recombinant DNA having a base sequence which codes for the mutein of bFGF in a culture meidum to produce and accumulate the mutein from the cultured broth.

17. A method as claimed in Claim 16, wherein further at least one constituent amino acid is replaced by other amino acid or at least one amino acid is added to the amino terminal.

18. A method as claimed in Claim 17, wherein at least one cysteine is replaced by a neutral amino acid.

Claims for the following Contracting States: ES,GR.

1. A method for producing mutein of basic fibroblast growth factor (bFGF), the mutein lacking 7 to 46 amino acids of the constituent amino acids of bFGF from carboxyl terminal, which comprises cultivating a transformant harboring a vector containing a recombinant DNA having a base sequence which codes for the mutein of bFGF in a culture meidum to produce and accumulate the mutein from the cultured broth.

2. A method as claimed in Claim 1, wherein further at least one constituent amino acid is replaced by other amino acid or at least one amino acid is added to the amino terminal.

3. A method as claimed in Claim 1, wherein the bFGF includes the amino acid sequence:
Phe-Phe-Leu-Arg-Ile-His-Pro-Asp-Gly-Arg-Val-Asp-Gly-Val-Arg-Glu-Lys-Ser-Asp-Pro
in its molecule.

4. A method as claimed in Claim 1, wherein the bFGF is human bFGF.

5. A method as claimed in Claim 2, wherein the constituent amino acid which is to be replaced is cysteine.

6. A method as claimed in Claim 2, wherein at least one cysteine is replaced by a neutral amino acid.

7. A method as claimed in Claim 6, wherein the neutral amino acid is serine or threonine.

8. A method as claimed in Claim 1, wherein the mutein is rhbFGF mutein CS23C129, in which amino acid residues at 130-(Lys) through 147-(Ser) positions of human bFGF are lacking and Cys at 70- and 88-positions are replaced by Ser.

9. A method as claimed in Claim 1, wherein the mutein is rhbFGF mutein CS23C137, in which amino acid residues at 138-(Ile) through 147-(Ser) positions of human bFGF are lacking, Cys at 70- and 88-positions are replaced by Ser, and Ile at 138-position is replcaed by Ser.

Figure 1

```
MetProAlaLeuProGluAspGlyGlySerGlyAlaPheProProGlyHisPheLysAsp
ATGCCAGCATTGCCCGAGGATGGCGGCAGCGGCGCCTTCCCGCCCGGCCACTTCAAGGAC

ProLysArgLeuTyrCysLysAsnGlyGlyPhePheLeuArgIleHisProAspGlyArg
CCCAAGCGGCTGTACTGCAAAAACGGGGGCTTCTTCCTGCGCATCCACCCCGACGGCCGA

ValAspGlyValArgGluLysSerAspProHisIleLysLeuGlnLeuGlnAlaGluGlu
GTTGACGGGGTCCGGGAGAAGAGCGACCCTCACATCAAGCTACAACTTCAAGCAGAAGAG

ArgGlyValValSerIleLysGlyValCysAlaAsnArgTyrLeuAlaMetLysGluAsp
AGAGGAGTTGTGTCTATCAAAGGAGTGTGTGCTAACCGTTACCTGGCTATGAAGGAAGAT

GlyArgLeuLeuAlaSerLysCysValThrAspGluCysPhePhePheGluArgLeuGlu
GGAAGATTACTGGCTTCTAAATGTGTTACGGATGAGTGTTTCTTTTTTGAACGATTGGAA

SerAsnAsnTyrAsnThrTyrArgSerArgLysTyrThrSerTrpTyrValAlaLeuLys
TCTAATAACTACAATACTTACCGGTCAAGGAAATACACCAGTTGGTATGTGGCACTGAAA

ArgThrGlyGlnTyrLysLeuGlySerLysThrGlyProGlyGlnLysAlaIleLeuPhe
CGAACTGGGCAGTATAAACTTGGATCCAAAACAGGACCTGGGCAGAAAGCTATACTTTTT

LeuProMetSerAlaLysSertrm
CTTCCAATGTCTGCTAAGAGCTGA
```

EP 0 326 907 A1

Figure 2

mcs : multi-cloning sites

E : EcoRI
B : BamHI
X : XbaI
P : PstI
H : HindIII

Plasmids expressing bFGF deleted COOH-terminal

pTB893 to pTB899

Figure 3

```
MetProAlaLeuProGluAspGlyGlySerGlyAlaPheProProGlyHisPheLysAsp
ATGCCAGCATTGCCCGAGGATGGCGGCAGCGGCGCCTTCCCGCCCGGCCACTTCAAGGAC

ProLysArgLeuTyrCysLysAsnGlyGlyPhePheLeuArgIleHisProAspGlyArg
CCCAAGCGGCTGTACTGCAAAAACGGGGGCTTCTTCCTGCGCATCCACCCCGACGGCCGA

ValAspGlyValArgGluLysSerAspProHisIleLysLeuGlnLeuGlnAlaGluGlu
GTTGACGGGGGTCCGGGAGAAGAGCGACCCTCACATCAAGCTACAACTTCAAGCAGAAGAG

ArgGlyValValSerIleLysGlyValCysAlaAsnArgTyrLeuAlaMetLysGluAsp
AGAGGAGTTGTGTCTATCAAAGGAGTGTGTGCTAACCGTTACCTGGCTATGAAGGAAGAT

GlyArgLeuLeuAlaSerLysCysValThrAspGluCysPhePhePheGluArgLeuGlu
GGAAGATTACTGGCTTCTAAATGTGTTACGGATGAGTGTTTCTTTTTTGAACGATTGGAA

Ser trm
TCCTAGCTAGCTAG
```

EP 0 326 907 A1

Figure 4

```
MetProAlaLeuProGluAspGlyGlySerGlyAlaPheProProGlyHisPheLysAsp        20
ATGCCAGCATTGCCCGAGGATGGCGGCAGCGGCGCCTTCCCGCCCGGCCACTTCAAGGAC        60


ProLysArgLeuTyrCysLysAsnGlyGlyPhePheLeuArgIleHisProAspGlyArg        40
CCCAAGCGGCTGTACTGCAAAAACGGGGGCTTCTTCCTGCGCATCCACCCCGACGGCCGA       120


ValAspGlyValArgGluLysSerAspProHisIleLysLeuGlnLeuGlnAlaGluGlu        60
GTTGACGGGGTCCGGGAGAAGAGCGACCCTCACATCAAGCTACAACTTCAAGCAGAAGAG       180


ArgGlyValValSerIleLysGlyValCysAlaAsnArgTyrLeuAlaMetLysGluAsp        80
AGAGGAGTTGTGTCTATCAAAGGAGTGTGTGCTAACCGTTACCTGGCTATGAAGGAAGAT       240


GlyArgLeuLeuAlaSerLysCysValThrAspGluCysPhePhePheGluArgLeuGlu       100
GGAAGATTACTGGCTTCTAAATGTGTTACGGATGAGTGTTTCTTTTTTGAACGATTGGAA       300


SerAsnAsnTyrAsn trm
TCTAATAACTACAAC TAG CTA GCTAG
```

EP 0 326 907 A1

Figure 5

```
MetProAlaLeuProGluAspGlyGlySerGlyAlaPheProProGlyHisPheLysAsp
ATGCCAGCATTGCCCGAGGATGGCGGCAGCGGCGCCTTCCCGCCCGGCCACTTCAAGGAC

ProLysArgLeuTyrCysLysAsnGlyGlyPhePheLeuArgIleHisProAspGlyArg
CCCAAGCGGCTGTACTGCAAAAACGGGGGCTTCTTCCTGCGCATCCACCCCGACGGCCGA

ValAspGlyValArgGluLysSerAspProHisIleLysLeuGlnLeuGlnAlaGluGlu
GTTGACGGGGTCCGGGAGAAGAGCGACCCTCACATCAAGCTACAACTTCAAGCAGAAGAG

ArgGlyValValSerIleLysGlyValCysAlaAsnArgTyrLeuAlaMetLysGluAsp
AGAGGAGTTGTGTCTATCAAAGGAGTGTGTGCTAACCGTTACCTGGCTATGAAGGAAGAT

GlyArgLeuLeuAlaSerLysCysValThrAspGluCysPhePhePheGluArgLeuGlu
GGAAGATTACTGGCTTCTAAATGTGTTACGGATGAGTGTTTCTTTTTTGAACGATTGGAA

SerAsnAsnTyrAsnThrTyrArgSerArgLysTyrThrSer trm
TCTAATAACTACAATACTTACCGGTCAAGGAAATACACCAGCTAG CTA GCTAG
```

EP 0 326 907 A1

Figure 6

```
MetProAlaLeuProGluAspGlyGlySerGlyAlaPheProProGlyHisPheLysAsp      20
ATGCCAGCATTGCCCGAGGATGGCGGCAGCGGCGCCTTCCCGCCCGGCCACTTCAAGGAC      60

ProLysArgLeuTyrCysLysAsnGlyGlyPhePheLeuArgIleHisProAspGlyArg      40
CCCAAGCGGCTGTACTGCAAAAACGGGGGCTTCTTCCTGCGCATCCACCCCGACGGCCGA     120

ValAspGlyValArgGluLysSerAspProHisIleLysLeuGlnLeuGlnAlaGluGlu      60
GTTGACGGGGTCCGGGAGAAGAGCGACCCTCACATCAAGCTACAACTTCAAGCAGAAGAG     180

ArgGlyValValSerIleLysGlyValCysAlaAsnArgTyrLeuAlaMetLysGluAsp      80
AGAGGAGTTGTGTCTATCAAAGGAGTGTGTGCTAACCGTTACCTGGCTATGAAGGAAGAT     240

GlyArgLeuLeuAlaSerLysCysValThrAspGluCysPhePhePheGluArgLeuGlu     100
GGAAGATTACTGGCTTCTAAATGTGTTACGGATGAGTGTTTCTTTTTTGAACGATTGGAA     300

SerAsnAsnTyrAsnThrTyrArgSerArgLysTyrThrSerTrpTyrValAla*trm*
TCTAATAACTACAATACTTACCGGTCAAGGAAATACACCAGTTGGTATGTGGCCTAGCTAGCTAG
```

EP 0 326 907 A1

Figure 7

MetProAlaLeuProGluAspGlyGlySerGlyAlaPheProProGlyHisPheLysAsp  20
ATGCCAGCATTGCCCGAGGATGGCGGCAGCGGCGCCTTCCCGCCCGGCCACTTCAAGGAC  60

ProLysArgLeuTyrCysLysAsnGlyGlyPhePheLeuArgIleHisProAspGlyArg  40
CCCAAGCGGCTGTACTGCAAAAACGGGGGCTTCTTCCTGCGCATCCACCCCGACGGCCGA  120

ValAspGlyValArgGluLysSerAspProHisIleLysLeuGlnLeuGlnAlaGluGlu  60
GTTGACGGGGTCCGGGAGAAGAGCGACCCTCACATCAAGCTACAACTTCAAGCAGAAGAG  180

ArgGlyValValSerIleLysGlyValCysAlaAsnArgTyrLeuAlaMetLysGluAsp  80
AGAGGAGTTGTGTCTATCAAAGGAGTGTGTGCTAACCGTTACCTGGCTATGAAGGAAGAT  240

GlyArgLeuLeuAlaSerLysCysValThrAspGluCysPhePhePheGluArgLeuGlu  100
GGAAGATTACTGGCTTCTAAATGTGTTACGGATGAGTGTTTCTTTTTTGAACGATTGGAA  300

SerAsnAsnTyrAsnThrTyrArgSerArgLysTyrThrSerTrpTyrValAlaLeuLys  120
TCTAATAACTACAATACTTACCGGTCAAGGAAATACACCAGTTGGTATGTGGCACTGAAA  360

ArgThrGly *trm*
CGAACTGGCTAGCTAGCTAG

Figure 8

```
MetProAlaLeuProGluAspGlyGlySerGlyAlaPheProProGlyHisPheLysAsp
ATGCCAGCATTGCCCGAGGATGGCGGCAGCGGCGCCTTCCCGCCCGGCCACTTCAAGGAC

ProLysArgLeuTyrCysLysAsnGlyGlyPhePheLeuArgIleHisProAspGlyArg
CCCAAGCGGCTGTACTGCAAAAACGGGGGCTTCTTCCTGCGCATCCACCCCGACGGCCGA

ValAspGlyValArgGluLysSerAspProHisIleLysLeuGlnLeuGlnAlaGluGlu
GTTGACGGGGTCCGGGAGAAGAGCGACCCTCACATCAAGCTACAACTTCAAGCAGAAGAG

ArgGlyValValSerIleLysGlyValCysAlaAsnArgTyrLeuAlaMetLysGluAsp
AGAGGAGTTGTGTCTATCAAAGGAGTGTGTGCTAACCGTTACCTGGCTATGAAGGAAGAT

GlyArgLeuLeuAlaSerLysCysValThrAspGluCysPhePhePheGluArgLeuGlu
GGAAGATTACTGGCTTCTAAATGTGTTACGGATGAGTGTTTCTTTTTTGAACGATTGGAA

SerAsnAsnTyrAsnThrTyrArgSerArgLysTyrThrSerTrpTyrValAlaLeuLys
TCTAATAACTACAATACTTACCGGTCAAGGAAATACACCAGTTGGTATGTGGCACTGAAA

ArgThrGlyGlnTyrLysLeuGlySer*trm*
CGAACTGGGCAGTATAAACTTGGATCCTAGCTAGCTAG
```

EP 0 326 907 A1

Figure 9

```
MetProAlaLeuProGluAspGlyGlySerGlyAlaPheProProGlyHisPheLysAsp        20
ATGCCAGCATTGCCCGAGGATGGCGGCAGCGGCGCCTTCCCGCCCGGCCACTTCAAGGAC        60

ProLysArgLeuTyrCysLysAsnGlyGlyPhePheLeuArgIleHisProAspGlyArg        40
CCCAAGCGGCTGTACTGCAAAAACGGGGGCTTCTTCCTGCGCATCCACCCCGACGGCCGA       120

ValAspGlyValArgGluLysSerAspProHisIleLysLeuGlnLeuGlnAlaGluGlu        60
GTTGACGGGGTCCGGGAGAAGAGCGACCCTCACATCAAGCTACAACTTCAAGCAGAAGAG       180

ArgGlyValValSerIleLysGlyValCysAlaAsnArgTyrLeuAlaMetLysGluAsp        80
AGAGGAGTTGTGTCTATCAAAGGAGTGTGTGCTAACCGTTACCTGGCTATGAAGGAAGAT       240

GlyArgLeuLeuAlaSerLysCysValThrAspGluCysPhePhePheGluArgLeuGlu       100
GGAAGATTACTGGCTTCTAAATGTGTTACGGATGAGTGTTTCTTTTTTGAACGATTGGAA       300

SerAsnAsnTyrAsnThrTyrArgSerArgLysTyrThrSerTrpTyrValAlaLeuLys       120
TCTAATAACTACAATACTTACCGGTCAAGGAAATACACCAGTTGGTATGTGGCACTGAAA       360

ArgThrGlyGlnTyrLysLeuGlySerLysThrGlyProGlyGlnLysAlaSer trm
CGAACTGGGCAGTATAAACTTGGATCCAAAACAGGACCTGGGCAGAAAGCTAGCTAGCTAG
```

EP 0 326 907 A1

Figure 10

# Figure 11

Figure 12

Figure 13

MetProAlaLeuProGluAspGlyGlySerGlyAlaPheProProGlyHisPheLysAsp
ATGCCAGCATTGCCCGAGGATGGCGGCAGCGGCGCCTTCCCGCCCGGCCACTTCAAGGAC

ProLysArgLeuTyrCysLysAsnGlyGlyPhePheLeuArgIleHisProAspGlyArg
CCCAAGCGGCTGTACTGCAAAAACGGGGGCTTCTTCCTGCGCATCCACCCCGACGGCCGA

ValAspGlyValArgGluLysSerAspProHisIleLysLeuGlnLeuGlnAlaGluGlu
GTTGACGGGGTCCGGGAGAAGAGCGACCCTCACATCAAGCTACAACTTCAAGCAGAAGAG

ArgGlyValValSerIleLysGlyVal⎡Ser⎤AlaAsnArgTyrLeuAlaMetLysGluAsp
AGAGGAGTTGTGTCTATCAAGGAGTG⎡AGC⎤GCTAATCGTTACCTGGCTATGAAGGAAGAT

GlyArgLeuLeuAlaSerLys⎡Ser⎤ValThrAspGluCysPhePhePheGluArgLeuGlu
GGAAGATTACTAGCTTCTAAA⎡TCT⎤GTTACGGATGAGTGTTTCTTTTTTGAACGATTGGAA

SerAsnAsnTyrAsnThrTyrArgSerArgLysTyrThrSerTrpTyrValAlaLeuLys
TCTAATAACTACAATACTTACCGGTCAAGGAAATACACCAGTTGGTATGTGGCACTGAAA

ArgThrGlyGlnTyrLysLeuGlySertrm
CGAACTGGGCAGTATAAACTTGGATCCTAGCTAGCTAG

EP 0 326 907 A1

Figure 14

```
MetProAlaLeuProGluAspGlyGlySerGlyAlaPheProProGlyHisPheLysAsp        20
ATGCCAGCATTGCCCGAGGATGGCGGCAGCGGCGCCTTCCCGCCCGGCCACTTCAAGGAC        60


ProLysArgLeuTyrCysLysAsnGlyGlyPhePheLeuArgIleHisProAspGlyArg        40
CCCAAGCGGCTGTACTGCAAAAACGGGGGCTTCTTCCTGCGCATCCACCCCGACGGCCGA       120


ValAspGlyValArgGluLysSerAspProHisIleLysLeuGlnLeuGlnAlaGluGlu        60
GTTGACGGGGTCCGGGAGAAGAGCGACCCTCACATCAAGCTACAACTTCAAGCAGAAGAG       180


ArgGlyValValSerIleLysGlyValSerAlaAsnArgTyrLeuAlaMetLysGluAsp        80
AGAGGAGTTGTGTCTATCAAAGGAGTGAGCGCTAATCGTTACCTGGCTATGAAGGAAGAT       240


GlyArgLeuLeuAlaSerLysSerValThrAspGluCysPhePhePheGluArgLeuGlu       100
GGAAGATTACTAGCTTCTAAATCTGTTACGGATGAGTGTTTCTTTTTTGAACGATTGGAA       300


SerAsnAsnTyrAsnThrTyrArgSerArgLysTyrThrSerTrpTyrValAlaLeuLys       120
TCTAATAACTACAATACTTACCGGTCAAGGAAATACACCAGTTGGTATGTGGCACTGAAA       360


ArgThrGlyGlnTyrLysLeuGlySerLysThrGlyProGlyGlnLysAlaSer Trm
CGAACTGGGCAGTATAAACTTGGATCCAAAACAGGACCTGGGCAGAAAGCTAGCTAGCTAG
```

# Fig. 15

# Fig. 16

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| E | EP-A-0 275 204 (AMGEN INC.)<br>* Page 3, lignes 7-48 * | 1 | C 12 N 15/00 |
| Y,P | WO-A-8 701 728 (BIOTECHNOLOGY RESEARCH PARTNERS)<br>* Page 61, ligne 1 - page 64, ligne 17 * | 1 | |
| Y | EP-A-0 237 966 (TAKEDA CHEMICAL INDUSTRIES)<br>* Page 21, ligne 35 - page 22, line 30 * | 1 | |
| A,D | PROC. NATL. ACAD. SCI. USA, vol. 82, October 1985, pages 6507-6511; F. ESCH et al.: "Primary structure of bovine pituitary basic fibroblast growth factor (FGF) and comparison with the amino-terminal sequence of bovine brain acidic FGF" | | |
| A,D | FEBS LETTERS, vol. 213, no. 1, March 1987, pages 189-194, Elsevier Science Publishers B.V.; K. TSUTOMU et al.: "Cloning and expression of cDNA encoding human basic fibroblast growth factor" | | TECHNICAL FIELDS SEARCHED (Int. Cl.4)<br><br>C 12 N |
| A | BIOCHEMICAL AND BIOPHYSICAL RESEARCH COMMUNICATIONS, vol. 144, no. 2, 29th April 1987, pages 543-550, Academic Press, Inc.; A. SOMMER et al.: "A form of human basic fibroblast growth factor with an extended amino terminus"<br>---                          -/- | | |

The present search report has been drawn up for all claims

| Place of search | Date of completion of the search | Examiner |
|---|---|---|
| THE HAGUE | 25-04-1989 | TURMO Y BLANCO C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,P | BIOCHEMICAL AND BIOPHYSICAL RESEARCH COMMUNICATIONS, vol. 151, no. 2, 15th March 1988, pages 701-708, Academic Press, Inc.; M. SENO et al.: "Stabilizing basic fibroblast growth factor using protein engineering" * Page 701; "Summary" * | 1 | |

TECHNICAL FIELDS SEARCHED (Int. Cl.4)

The present search report has been drawn up for all claims

| Place of search | Date of completion of the search | Examiner |
|---|---|---|
| THE HAGUE | 25-04-1989 | TURMO Y BLANCO C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)